(19) Europäisches Patentamt / European Patent Office / Office européen des brevets

(11) EP 3 810 075 B1

(12) **FASCICULE DE BREVET EUROPEEN**

(45) Date de publication et mention de la délivrance du brevet:
**28.06.2023 Bulletin 2023/26**

(21) Numéro de dépôt: **19731740.7**

(22) Date de dépôt: **20.06.2019**

(51) Classification Internationale des Brevets (IPC):
***A61K 8/41*** *(2006.01)* ***A61Q 5/10*** *(2006.01)*
***A61K 8/73*** *(2006.01)* ***A61K 8/04*** *(2006.01)*

(52) Classification Coopérative des Brevets (CPC):
**A61K 8/73; A61K 8/046; A61K 8/41; A61Q 5/10**

(86) Numéro de dépôt international:
**PCT/EP2019/066361**

(87) Numéro de publication internationale:
**WO 2019/243505 (26.12.2019 Gazette 2019/52)**

(54) **DISPOSITIF DE DISTRIBUTION D'UN PRODUIT DE COLORATION CAPILLAIRE METTANT EN OEUVRE UNE COMPOSITION COLORANTE ET UNE COMPOSITION OXYDANTE COMPRENANT UNE GOMME DE SCLEROGLUCANE.**

VORRICHTUNG ZUR ABGABE EINES HAARFÄRBEMITTELS UNTER VERWENDUNG EINER FÄRBEMITTELZUSAMMENSETZUNG UND EINER OXIDIERENDEN ZUSAMMENSETZUNG MIT EINEM SCLEROGLUCANGUMMI

DEVICE FOR DISPENSING A HAIR DYEING PRODUCT USING A DYE COMPOSITION AND AN OXIDIZING COMPOSITION COMPRISING A SCLEROGLUCAN GUM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.06.2018 FR 1855427**

(43) Date de publication de la demande:
**28.04.2021 Bulletin 2021/17**

(73) Titulaire: **L'OREAL**
**75008 Paris (FR)**

(72) Inventeurs:
- **MULLER, Sabrina**
  **93400 SAINT-OUEN (FR)**
- **CHARRIER, Delphine**
  **93400 SAINT-OUEN (FR)**
- **PIZZINO, Aldo**
  **93400 SAINT-OUEN (FR)**
- **SIMONET, Frédéric**
  **93400 SAINT-OUEN (FR)**
- **YADEL, Cindy**
  **93400 SAINT-OUEN (FR)**
- **CARDONNEL, Fanny**
  **93400 SAINT-OUEN (FR)**
- **MILIC, Mladen**
  **93400 SAINT-OUEN (FR)**

(74) Mandataire: **L'Oreal**
**Service D.I.P.I.**
**9, rue Pierre Dreyfus**
**92110 Clichy (FR)**

(56) Documents cités:
**US-A1- 2010 192 969** **US-A1- 2016 279 036**
**US-A1- 2017 354 584**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Processed by Luminess, 75001 PARIS (FR)

## Description

La présente invention a pour objet un dispositif de distribution selon la revendication 1 d'un produit de coloration des fibres kératiniques comprenant une composition colorante (A) comprenant un ou plusieurs colorant(s) d'oxydation, et un ou plusieurs agents alcalins et une composition oxydante (B) comprenant un ou plusieurs agent(s) oxydant(s) chimique(s) , l'une au moins des compositions (A) et/ou (B) comprenant une ou plusieurs gommes de scléroglucane de préférence en une teneur totale en poids supérieure ou égale à 0,5% par rapport au poids total de la (des) composition(s) (A) et/ou (B) la(les) contenant ainsi qu'un procédé de coloration des fibres kératiniques mettant en oeuvre ledit dispositif.

La présente invention a trait au domaine de la coloration des fibres kératiniques et plus particulièrement à celui de la coloration capillaire, en particulier de la coloration d'oxydation.

Parmi les méthodes de coloration des fibres kératiniques humaines, telles que les cheveux, on peut citer la coloration d'oxydation ou permanente. Plus particulièrement, ce mode de coloration met en oeuvre un ou plusieurs colorants d'oxydation, habituellement une ou plusieurs bases d'oxydation éventuellement associées à un ou plusieurs coupleurs.

La variété des molécules mises en jeu au niveau des bases d'oxydation et des coupleurs permet l'obtention d'une riche palette de couleurs.

Les procédés de coloration d'oxydation consistent donc à employer avec ces compositions tinctoriales, une composition comprenant au moins un agent oxydant, en général le peroxyde d'hydrogène, en condition de pH alcalin dans la grande majorité des cas. Cet agent oxydant a pour rôle de révéler la coloration, par une réaction de condensation oxydative des colorants d'oxydation entre eux.

Ces compositions, en particulier les compositions comprenant des colorants d'oxydation, sont évidemment sensibles à l'oxydation et contiennent de ce fait des agents réducteurs ou antioxydants. Cette action de protection contre l'oxydation est en outre renforcée grâce à l'atmosphère inerte qui est parfois employée lors du conditionnement de ces compositions.

La difficulté rencontrée avec ce type de compositions résulte justement de leur sensibilité à l'oxydation. En effet, lors de leur utilisation, elles sont mises en contact avec l'oxygène de l'air et cela nécessite alors qu'elles soient utilisées rapidement. Dans le cas contraire, les compositions deviennent après stockage inutilisables et sont perdues.

Il existe dans le domaine de la coloration capillaire, des compositions alcalines, en particulier colorantes, et oxydantes qui sont conditionnées séparément dans un même récipient pressurisé et qui permettent d'éviter la mise en contact de la composition avec l'air lors de leur utilisation. Ces compositions peuvent se présenter sous forme de mousse ou de crème. US 2010/192969 A1 (DEGEORGE MICHAEL [US] ET AL) 5 août 2010 décrit une composition de coloration capillaire comprenant un agent épaississant.

Cependant, la puissance de la coloration ainsi que la couverture des cheveux, en particulier des cheveux blancs, restent encore à améliorer ainsi que les propriétés rhéologiques du ou des produit(s) issu(s) du récipient pressurisé, notamment au niveau de la facilité de mélange des compositions alcalines et oxydantes, de l'homogénéité du mélange et de la qualité d'application sur les fibres kératiniques (en particulier au niveau du caractère mouillant, de l'adhérence aux racines et de la facilité d'allongement du mélange le long des mèches de cheveux).

Il existe un besoin constant de développer des dispositifs permettant de délivrer des compositions de coloration tout en conservant les valeurs d'usage.

La coloration d'oxydation doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agressions extérieures telles que la lumière, les intempéries, le lavage, les ondulations permanentes, la transpiration et les frottements.

Le procédé de coloration doit également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (c'est-à-dire abîmées) de sa pointe à sa racine, afin d'obtenir une coloration la plus homogène possible des fibres kératiniques. Les compositions de coloration doivent également permettre de conférer de bonnes propriétés cosmétiques aux fibres kératiniques, en particulier du soin, de la douceur et/ou de la discipline, présenter de bonnes qualités d'usage, en particulier être faciles à appliquer, tout en permettant d'atteindre des résultats couleur visibles (c'est-à-dire notamment intenses, chromatiques), homogènes et tenaces.

Les compositions mises en oeuvre dans un procédé de coloration doivent, en outre, présenter de bonnes propriétés de mélange et d'application sur les fibres kératiniques, et notamment de bonnes propriétés rhéologiques pour ne pas couler, lors de leur application, sur le visage, le cuir chevelu, ou en dehors des zones que l'on se propose de teindre, cela permet notamment une application homogène des racines jusqu'au pointes.

En particulier on cherche à obtenir des compositions de coloration ou oxydantes stables dans le temps pendant plusieurs semaines. Par « stable » au sens de la présente invention on entend en particulier que des caractéristiques physiques telles que l'aspect, le pH et/ou la viscosité varient peu, voire ne varient pas, au court du temps, en particulier,

que la viscosité de la composition n'évolue pas ou peu pendant le stockage et/ou que la composition ne déphase pas pendant le stockage.

En effet, il est souhaitable que les compositions de coloration ou oxydantes soient stables dans le temps, en particulier stables après 1 mois à 45°C, voire après 2 mois à 45°C.

On cherche également à obtenir des compositions de coloration stables sur une large gamme de pH et en particulier vis-à-vis de pH extrêmes, par exemple à des pH alcalins allant de 9 à 12. Enfin, les compositions de coloration peuvent parfois être déstabilisées (déphasées) par des teneurs élevés en certains composés, comme par exemple en colorants d'oxydation et/ou des composés cationiques tels que des polymères cationiques, et il est donc souhaitable que ces compositions soient stables dans ces conditions, en particulier qui ne déphase pas.

Ainsi l'un des objectifs de la présente invention est de proposer un procédé de coloration des fibres kératiniques, de préférence des fibres kératiniques humaines telles que les cheveux qui ne présentent pas les inconvénients mentionnés ci-dessus,

Ces buts et d'autres sont atteints par la présente invention qui a donc pour objet un dispositif de distribution d'un produit de coloration des fibres kératiniques constitué d'un récipient, de préférence sous forme d'aérosol comprenant:

i) au moins deux compartiments (a) et (b) séparés l'un de l'autre, le compartiment (a) comprenant une composition (A) comprenant :

- un ou plusieurs colorant(s) d'oxydation ;
- un ou plusieurs agents alcalins ; choisis selon la revendication 1 et
  le compartiment (b) comprenant une composition oxydante B comprenant ;
- un ou plusieurs agent(s) oxydants chimiques, de préférence choisi(s) parmi le peroxyde d'hydrogène et/ou un ou plusieurs système(s) générateur(s) de peroxyde d'hydrogène, de préférence le peroxyde d'hydrogène ; et
- l'une et/ou l'autre des compositions (A) et/ou (B) comprenant une ou plusieurs gommes de scléroglucane, de préférence en une teneur totale en poids supérieure ou égale à 0,5% par rapport au poids total de la (des) composition(s) (A) et/ou (B) la (les) contenant ;

ii)un moyen de distribution muni d'au moins un orifice de distribution, en communication avec les compartiments (a) et (b), permettant de délivrer de manière simultanée les compositions (A) et (B), à l'état mélangé ou séparé.

De préférence les compositions (A) et (B) comprennent une ou plusieurs gommes de scléroglucane, de préférence en une teneur totale en poids supérieure ou égale à 0,5% par rapport au poids total des composition(s) (A) et (B) les contenant.

L'invention a également pour objet un procédé de coloration des fibres kératiniques, de préférence humaines, mettant en oeuvre ce dispositif.

Plus précisément, l'invention pour objet un procédé de coloration des fibres kératiniques, de préférence des fibres kératiniques humaines telles que les cheveux, mettant en oeuvre l'application sur les fibres:

a) d'une composition colorante (A) comprenant :

- un ou plusieurs colorant(s) d'oxydation ;
- un ou plusieurs agents alcalins choisis selon la revendication 1 ; et

b) d'une composition oxydante (B) comprenant :

- un ou plusieurs agent(s) oxydants chimiques, de préférence choisi parmi le peroxyde d'hydrogène et/ou un ou plusieurs système(s) générateur(s) de peroxyde d'hydrogène, de préférence parmi le peroxyde d'hydrogène ; et

l'une et/ou l'autre des compositions (A) et/ou (B) comprenant une ou plusieurs gommes de scléroglucane, de préférence en une teneur totale en poids supérieure ou égale à 0,5% par rapport au poids total de la (des) composition(s) (A) et/ou (B) la (les) contenant ;
les compositions (A) et (B) étant conditionnées dans un dispositif constitué d'un récipient, de préférence pressurisé, comprenant au moins deux compartiments (a) et (b) séparés l'un de l'autre, le compartiment (a) comprenant la composition (A) et le compartiment (b) comprenant la composition (B), le dispositif comprenant un moyen de distribution muni d'au moins un orifice de distribution, en communication avec les compartiments (a) et (b), permettant de délivrer les compositions (A) et (B) de manière simultanée.

Au sens de la présente invention, par « composition pour la coloration » ou par « composition colorante », on entend une composition destinée à être appliquée sur les fibres kératiniques, de préférence les fibres kératiniques humaines et en particulier les cheveux, en particulier après mélange avec une composition oxydante (B) telle que définie précédemment.

Au sens de la présente invention, par « composition colorante prête à l'emploi » ou « composition prête à l'emploi », on entend une composition résultant du mélange d'une composition colorante et d'une composition oxydante, destinée à être appliquée immédiatement sur les fibres kératiniques. La composition colorante prête à l'emploi est avantageusement préparée juste avant l'application sur lesdites fibres kératiniques.

Le dispositif et le procédé de l'invention permettent d'obtenir de bonnes propriétés tinctoriales des fibres kératiniques, notamment en termes de montée, d'intensité, de chromaticité et/ou de sélectivité. Elles permettent également d'obtenir des compositions ayant de bonnes propriétés rhéologiques pour ne pas couler, lors de leur application, sur le visage, le cuir chevelu, ou en dehors des zones que l'on se propose de teindre.

Les compositions mises en oeuvre dans le procédé selon l'invention sont stables. Par « stable » au sens de la présente invention on entend en particulier que des caractéristiques physiques telles que l'aspect, le pH et/ou la viscosité varient peu, voire ne varient pas, au court du temps, en particulier, que la viscosité de la composition n'évolue pas ou peu pendant le stockage et/ou que la composition ne déphase pas au cours du stockage. En particulier, il est souhaitable que les compositions de coloration soient stables dans les temps, en particulier stables après 1 mois à 45°C, voire après 2 mois à 45°C.

De plus les compositions mises en oeuvre selon l'invention présentent l'avantage d'être stables (ne déphasent pas) indépendamment du pH et en particulier vis-à-vis de pH extrêmes (par exemple à des pH alcalins allant de 9 à 12). Enfin, les compositions sont de préférence stables (ne déphasent pas) même en présence d'une teneur importante en certains composés, comme par exemple en colorants d'oxydation et/ou en composés cationiques, tels que des polymères cationiques.

Par ailleurs, les compositions mises en oeuvre dans le dispositif et le procédé selon l'invention sont de façon avantageuse translucides, ce qui leur confère un aspect visuel esthétique et attrayant pour le consommateur, de même que la composition prête à l'emploi résultant du mélange des compositions colorantes (A) et oxydante (B), lors de la mise en oeuvre du procédé selon l'invention.

Cette composition de coloration translucide prête à l'emploi offre la possibilité de visualiser l'évolution du résultat couleur pendant le temps de pause du produit laissant le choix de stopper au moment où le résultat lui convient.

Le dispositif comprenant les compositions conduit également à de bonnes qualités d'usage, en particulier l'application des compositions (A) et (B) est facile et la répartition est homogène le long des fibres kératiniques.

Le dispositif et le procédé de l'invention conduisent également à de bonnes propriétés cosmétiques, notamment en terme de démêlage, souplesse et de douceur des cheveux.

En outre les compositions (A) et (B) mises en oeuvre selon l'invention se mélangent facilement, préalablement à l'application sur les fibres kératiniques ou directement sur les fibres kératiniques lors d'une application simultanée des compositions (A) et (B) sur les cheveux.

De plus, grâce à leurs propriétés rhéologiques très stables et proches le compositions colorantes et oxydantes mises en oeuvre permettent l'utilisation d'un conditionnement dans le dispositif selon l'invention pour la mise en oeuvre du procédé selon l'invention

En effet, dans le cadre de la mise en oeuvre d'un dispositif de distribution selon l'invention, il est nécessaire que les viscosités des compositions (A) et (B) soient stables, et de préférence proches, de façon à ce que la quantité de composition (A) et (B) délivrées de façon simultanée reste stable dans le temps, ce qui ne pourrait être le cas si la viscosité de l'une des compositions s'avérait ne pas être stable. De plus, le mélange se faisant lors de la distribution ou directement sur les fibres kératiniques lors de l'application, il est nécessaire que les compositions se mélangent aisément.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine, notamment dans les expressions « compris entre » et « allant de... à ... » et « variant de ... à ... » .

Les fibres kératiniques sont de préférence des fibres kératiniques humaines,de préférence les cheveux.

L'expression « *au moins un* » est équivalente à l'expression « *un ou plusieurs ».*

Avantageusement, les compositions colorantes et oxydantes mises en oeuvre dans le procédé selon l'invention présentent une texture épaissie, sous forme de crème ou de gel, et sont de préférence translucide.

Les compositions (A) et (B) mises en oeuvre dans le procédé et le dispositif selon l'invention présentent généralement à température ambiante une viscosité supérieure à 50 cps, de préférence comprise entre 200 et 100000 cps, plus préférentiellement entre 400 et 50000 cps, et encore plus préférentiellement entre 500 à 10000 cps, mieux de 600 à 8000 cps. Cette viscosité est mesurée à 25°C à une vitesse de rotation de 200 tours/mn à l'aide d'un rhéomètre tel que le rheomat RM 180 équipé d'un mobile n° 3 ou 4, la mesure étant effectuée après 60 secondes de rotation du mobile (temps au bout duquel on observe une stabilisation de la viscosité et de la vitesse de rotation du mobile.

Selon un mode de réalisation particulier, la viscosité de chacune des deux compositions est comprise entre entre 500 à 10000 cps, mieux de 600 à 8000 cps.

***Composition colorante (A)***

***Colorants d'oxydation***

La composition colorante (A) selon l'invention comprend un ou plusieurs colorants d'oxydation.

Les précurseurs de colorant d'oxydation utilisables dans la présente invention sont en général choisis parmi les bases d'oxydation, éventuellement combinées à un ou plusieurs coupleurs.

Les bases d'oxydation peuvent être de préférence choisies parmi les paraphénylènediamines, les bis-phénylalkylènediamines, les para-aminophénols, les ortho-aminophénols, les bases hétérocycliques et leurs sels d'addition.

Préférentiellement, la ou les bases d'oxydation de l'invention sont choisies parmi les paraphénylènediamines et les bases hétérocycliques. Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino-N,N-diéthyl-3-méthylaniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino-2-chloroaniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2 hydroxyméthyl paraphénylènediamine, la 2-méthoxyméthyl paraphénylènediamine, la N,N-diméthyl-3-méthyl paraphénylènediamine, la N,N-(éthyl,-β-hydroxyéthyl) paraphénylènediamine, la N-(β,y-dihydroxypropyl) paraphénylènediamine, la N-(4' aminophényl) paraphénylènediamine, la N-phényl paraphénylènediamine, la 2-β- hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, la 2-méthoxyméthyl paraphénylènediamine et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl)-N,N'-bis-(4'-aminophényl)éthylènediamine, la N,N'-bis-(4-aminophényl) tétraméthylènediamine, la N,N'-bis-(β-hydroxyéthyl)-N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3' méthylphényl) éthylènediamine, le 1,8-bis-(2,5-diaminophénoxy)-3,6-dioxaoctane, et leurs sels d'addition.

Parmi les para-aminophénols, on peut citer à titre d'exemple, le para-amino phénol, le 4-amino-3-méthyl phénol, le 4-amino-3-fluoro phénol, le 4-amino-3-chlorophénol, le 4-amino-3-hydroxyméthyl phénol, le 4-amino-2-méthyl phénol, le 4-amino-2-hydroxyméthyl phénol, le 4-amino-2-méthoxyméthyl phénol, le 4-amino-2-aminométhyl phénol, le 4-amino 2-(β-hydroxyéthyl aminométhyl) phénol, le 4-amino-2-fluoro phénol, et leurs sels d'addition avec un acide.

Parmi les ortho-aminophénols, on peut citer à titre d'exemple, le 2-amino phénol, le 2-amino-5-méthyl phénol, le 2-amino-6-méthyl phénol, le 5-acétamido-2- amino phénol, et leurs sels d'addition.

Parmi les bases hétérocycliques, on peut en particulier les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5 diamino pyridine, la 2-(4-méthoxyphényl)amino 3 amino pyridine, la 3,4-diamino pyridine, et leurs sels d'addition.

D'autres bases d'oxydation pyridiniques utiles dans la présente invention sont les bases d'oxydation 3-amino pyrazolo-[1,5 a]-pyridines ou leurs sels d'addition décrits par exemple dans la demande de brevet FR 2801308.

A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine; l'acide 3-aminopyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-aminopyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-aminopyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-aminopyrazolo[1,5-a]pyridine-7-ol ; le 2-β-hydroxyéthoxy-3-aminopyrazolo[1,5-a]pyridine; le 2-(4-diméthylpypérazinium-1-yl)-3-aminopyrazolo[1,5-a]pyridine; ainsi que leurs sels d'addition.

Plus particulièrement les bases d'oxydation selon l'invention sont choisies parmi les 3-aminopyrazolo-[1,5 a]-pyridines de préférence substituées en position 2 par :

a) un groupe (di)($C_1$-$C_6$)(alkyl)amino les groupes alkyles pouvant être substitués par un ou plusieurs groupes hydroxy, amino, ou imidazolium ;

b) un groupe hétérocycloalkyle comprenant de 5 à 7 chainons, et de 1 à 3 hétéroatomes, cationique ou non, éventuellement substitué par un ou plusieurs groupes ($C_1$-$C_6$)alkyle tel que di(Ci-$C_4$)alkylpipérazinium ;

c) un groupe ($C_1$-$C_6$)alkoxy éventuellement substitué par un ou plusieurs groupes hydroxy tel que β-hydroxyalkoxy ainsi que leurs sels d'addition.

Parmi les dérivés pyrimidiniques, on peut citer les composés décrits par exemple dans les brevets DE 2359399 ; JP 88 169571 ; JP 05-63124 ; EP 0770375 ou demande de brevet WO 96/15765 comme la 2,4,5,6-tétra-aminopyrimidine, la 4 hydroxy 2,5,6-triaminopyrimidine, la 2 hydroxy 4,5,6-triaminopyrimidine, la 2,4 dihydroxy 5,6-diaminopyrimidine, la 2,5,6 triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5 diamino 1-méthyl pyrazole, le 4,5-diamino 1-(β-hydroxyéthyl) pyrazole, le 3,4-diamino pyrazole, le 4,5-diamino-1- (4'-chlorobenzyl) pyrazole, le 4,5-diamino-1,3-diméthyl pyrazole, le 4,5-diamino-3-méthyl-1-phényl pyrazole, le 4,5-diamino-1-méthyl-3-phényl pyrazole, le 4-amino-1,3-diméthyl-5-hydrazino pyrazole, le 1-benzyl-4,5-diamino-3-méthyl pyrazole, le 4,5-diamino-3-tert-butyl-1-méthyl pyrazole, le 4,5-diamino-1-tert-butyl 3-méthyl pyrazole, le 4,5-diamino-1-(β-hydroxyéthyl)-3-méthyl pyrazole, le 4,5-diamino-1-éthyl-3-méthyl pyrazole, le 4,5-diamino-1-éthyl-3-(4'-méthoxyphényl) pyrazole, le 4,5-diamino-1-éthyl 3-hydroxyméthyl pyrazole, le 4,5-diamino-3-hydroxyméthyl-1-méthyl pyrazole, le 4,5-diamino-3-hydroxyméthyl-1-isopropyl pyrazole, le 4,5-diamino-3-méthyl-1-isopropyl pyrazole, le 4-amino-5-(2'-aminoéthyl)amino-1,3-diméthyl pyrazole, le 3,4,5-triamino pyrazole, le 1-méthyl-3,4,5-triamino pyrazole, le 3,5-diamino-1-méthyl-4-méthylamino pyrazole, le 3,5-diamino-4-(β-hydroxyéthyl)amino-1-méthyl pyrazole, et leurs sels d'addition. De préférence les bases d'oxydation hétérocycliques de l'invention sont choisies parmi les 4,5-diaminopyrazoles telle que le 4,5-diamino-1-(β-hydroxyéthyl) pyrazole. On peut aussi utiliser le 4-5-diamino-1-(β-méthoxyéthyl)pyrazole.

De préférence, on utilisera un 4,5-diaminopyrazole et encore plus préférentiellement le 4,5-diamino-1-(β-hydroxyéthyl)-pyrazole et/ou l'un de ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,SH-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

La ou les bases d'oxydation utilisées dans le cadre de l'invention sont en général présentes en quantité allant de 0,001 à 10 % en poids environ du poids total de la composition colorante, de préférence allant de 0,005 à 5 %.

Les coupleurs additionnels conventionnellement utilisés pour la teinture de fibres kératiniques sont de préférence choisis parmi les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro-1,3-dihydroxy benzène, le 1-hydroxy-3-aminobenzène, le 2-méthyl-5-aminophénol, le 3-amino-2-chloro-6-méthyl phénol, le 2-méthyl-5-hydroxyéthylaminophénol, le 2,4-diamino-1-(β-hydroxyéthyloxy) benzène, le 2-amino-4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le thymol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H-3-méthyl pyrazole-5-one, la 1-phényl-3-méthyl pyrazole 5-one, le 2,6-diméthyl pyrazolo-[1,5-b]-1,2,4-triazole, le 2,6-diméthyl-[3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

Dans le cadre de la présente invention, lorsqu'ils sont présents, le ou les coupleurs sont généralement présents en quantité totale allant de 0,001 à 10 % en poids environ du poids total de la composition colorante, de préférence allant de 0,005 à 5 % en poids par rapport au poids total de la composition colorante (A).

De préférence, la teneur totale en colorants d'oxydation dans la composition selon l'invention est comprise entre 0,001 à 20% en poids ; de préférence entre 0,001% et 10% en poids, de préférence entre 0,01% et 5% en poids, par rapport au poids de la composition colorante (A).

### *Gommes de scléroglucane*

Selon l'invention, la composition (A) comprend de préférence une ou plusieurs gommes de scléroglucane, de préférence en une teneur totale supérieure ou égale à 0,5% en poids par rapport au poids de la composition (A).

Les gommes de scléroglucane sont des polysaccharides d'origine microbienne produits par un champignon de type Sclerotium, en particulier Sclerotium rolfsii. Ce sont des polysaccharides constitués uniquement de motifs glucose.

Les gommes de scléroglucane peuvent être modifiées ou non. De préférence les gommes de scléroglucane utilisées dans la présente invention sont non modifiées.

Des exemples de gommes de scléroglucane utilisables dans la présente invention sont, de manière non limitative, les produits vendus sous la dénomination ACTIGUM CS, en particulier ACTIGUM CS 11, par la société SANOFI BIO INDUSTRIES et sous la dénomination AMIGUM ou AMIGEL par la société ALBAN MULLER INTERNATIONAL.

D'autres gommes de scléroglucane, telles que celle traitée au glyoxal décrite dans la demande de brevet français n° 2 633 940, peuvent également être utilisées.

Lorsque la composition (A) comprend une ou plusieurs gomme(s) de scléroglucane, leur teneur totale représente de préférence de 0,5 à 10 % en poids, plus préférentiellement de 0,5 à 5 % en poids, et encore plus préférentiellement de 0,5 à 3 % en poids, mieux de 0,5% à 2% en poids et encore plus préférentiellement de 0,7 à 1,5 %en poids, par rapport au poids total de la composition (A).

Dans un mode de réalisation préféré de l'invention, la composition (A) comprend une ou plusieurs gommes de scléroglucane, de préférence en une teneur totale supérieure ou égale à 0,5% en poids par rapport au poids de la composition (A).

Plus préférentiellement, la composition (A) comprend une ou plusieurs gomme(s) de scléroglucane en une teneur totale supérieure ou égale à 0,5%, de préférence de 0,5 à 10 % en poids, plus préférentiellement de 0,5 à 5 % en poids, et encore plus préférentiellement de 0,5 à 3 % en poids, mieux de 0,5% à 2% en poids et encore plus préférentiellement de 0,7 à 1,5 %en poids, par rapport au poids total de la composition (A)

### Agents alcalins

La composition (A) mise en oeuvre dans le procédé selon l'invention comprend un ou plusieurs agents alcalins. Le ou les agents alcalins (également appelés agents alcalinisants) peuvent être minéraux, organiques et/ou hybrides, en particulier minéraux et/ou organiques.

Selon un premier mode de réalisation avantageux de l'invention, le ou les agent(s) alcalin(s) sont choisis parmi le ou les agents alcalins minéraux, de préférence choisis parmi l'ammoniaque, encore appelé hydroxyde d'ammonium, (ou agents précurseurs d'ammoniac comme les sels d'ammonium par exemple les halogénures d'ammonium et en particulier le chlorure d'ammonium), les silicates, les phosphates, les carbonates ou bicarbonates de métaux alcalins ou alcalino-terreux, tels que métasilicates de métaux alcalins ou alcalino-terreux, les carbonates ou bicarbonates de sodium ou de potassium, les hydroxydes de sodium ou de potassium, ou leurs mélanges.

selon ce mode de réalisation, les agents alcalins sont choisis parmi l'ammoniaque (ou agents précurseurs d'ammoniac comme les sels d'ammonium par exemple les halogénures d'ammonium et en particulier le chlorure d'ammonium) et/ou les métasilicates. de métaux alcalins ou alcalino-terreux

Selon un second mode de réalisation avantageux de l'invention, le ou les agent(s) alcalin(s) sont choisis parmi le ou les agents alcalins organiques ,de préférence choisis parmi les amines organiques dont le $pK_b$ à 25°C est inférieur

à 12, et de préférence inférieur à 10, encore plus avantageusement inférieur à 6. Il est à noter qu'il s'agit du $pK_b$ correspondant à la fonction de basicité la plus élevée. En outre, les amines organiques ne comprennent pas de chaîne grasse, alkyle ou alcényle, comprenant plus de dix atomes de carbone.

Selon ce mode de réalisation le ou les agents alcalins organiques sont choisis parmi les alcanolamines et/ou les acides aminés.

Selon un premier mode de réalisation préféré, le ou les agents alcalins sont choisis parmi les alcanolamines.

Par alcanolamine on entend une amine organique comprenant une fonction amine primaire, secondaire ou tertiaire, et un ou plusieurs groupements alkyle, linéaires ou ramifiés, en $C_1$ à $C_8$ porteurs d'un ou plusieurs radicaux hydroxy.

Conviennent en particulier à la réalisation de l'invention les amines organiques choisies parmi les alcanolamines telles que les mono-, di- ou tri-alcanolamines, comprenant un à trois radicaux hydroxyalkyle, identiques ou non, en $C_1$ à $C_4$.

Les composés de ce type, sont de préférence choisis parmi la monoéthanolamine (MEA), la diéthanolamine, la triéthanolamine, la monoisopropanolamine, la diisopropanolamine, la N,N-diméthyléthanolamine, le 2-amino-2-méthyl-1-propanol, la triisopropanol-amine, le 2-amino-2-méthyl-1,3-propanediol, le 3-amino-1,2-propancdiol, le 3-diméthylamino-1,2-propanediol, le tris-hydroxyméthylamino-méthane, et leurs mélanges, de préférence la monoéthanolamine (MEA).

Selon un second mode de réalisation préféré, le ou les agents alcalins sont choisis parmi les acides aminés.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

A titre d'acides aminés utilisables dans la composition selon la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

De manière avantageuse, les acides aminés sont choisis parmi les acides aminés basiques, notamment comprenant une fonction amine supplémentaire éventuellement incluse dans un cycle ou dans une fonction uréido.

De tels acides aminés basiques sont choisis de préférence parmi l'histidine, la lysine, l'arginine, l'ornithine, la citrulline.

Selon un mode de réalisation particulièrement préféré de l'invention, la composition (A) comprend :

- un ou plusieurs agent(s) alcalin(s) minéraux, de préférence choisis parmi l'ammoniaque et/ou les métasilicates de métaux alcalins ou alcalinoterreux de préférence l'ammoniaque; et
- un ou plusieurs agents alcalins organiques, de préférence choisis parmi les alcanolamines et/ou les acides aminés, de préférence parmi les alcanolamines, de préférence la monoéthanolamine.

La composition (A) selon l'invention comprend de préférence un ou plusieurs agent(s) alcalin(s) minéral(aux) et un ou plusieurs agent(s) alcalin(s) choisi(s) parmi les alcanolamine(s).

Lorsque la composition (A) comprend de l'ammoniaque (hydroxyde d'ammonium), sa teneur va de préférence de 0,1 à 10% en poids, plus préférentiellement de 0,5 à 8% en poids, mieux de 1 à 6% en poids par rapport au poids total de la composition (A).

Lorsque la composition (A) comprend une ou plusieurs alcanolamines, leur teneur totale va de préférence de 0,5 à 10% en poids, plus préférentiellement de 1 à 9% en poids, mieux de 2 à 8% en poids par rapport au poids total de la composition(A).

De façon préférée la composition colorante (A) selon l'invention comprend une teneur totale en agents alcalins allant de 1 à 20% en poids, plus préférentiellement de 3 à 18% en poids, mieux de 5 à 16 % en poids par rapport au poids total de la composition (A).

**Polymères Associatif**

La composition colorante (A) selon l'invention peut également comprendre un ou plusieurs polymères associatifs. Les polymères associatifs selon l'invention sont des polymères comprenant au moins une chaîne grasse en $C_8$ - $C_{30}$ et dont les molécules sont capables, dans le milieu de formulation, de s'associer entre elles ou avec des molécules d'autres composés.

De préférence, la chaîne grasse comporte de 10 à 30 atomes de carbone.

Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes (comprenant au moins une chaîne grasse) par lesquelles les polymères interagissent et se rassemblent entre eux ou

avec d'autres molécules.

Les *polymères associatifs pouvant être utilisés dans la composition selon* l'invention peuvent être choisis parmi les polymères associatifs non ioniques, anioniques, cationiques et amphotères, et leurs mélanges.

De façon particulièrement préférée, le ou les polymères associatifs sont non ioniques, et de préférence choisis parmi les celluloses modifiées par des groupements comportant au moins une chaîne grasse.
De préférence, le ou les polymères associatifs non ioniques sont choisis parmi les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, et les hydroxyéthylcelluloses modifiées par des groupes polyalkylène glycol éther d'alkyl phénol, et leurs mélanges, de préférence la cétylhydroxyéthylcellulose.

***Polymères cationiques***

Selon un mode de réalisation avantageux de l'invention, la composition (A) comprend un ou plusieurs polymères cationiques, différents des polymères fixants cationiques cités précédemment, et différents des polymères associatifs cationiques cités précédemment.

Comme polymères cationiques utilisables dans les compositions selon l'invention, on peut citer en particulier :

(1) les cyclopolymères d'alkyl diallyl amine ou de dialkyl diallyl ammonium tels que les homopolymères ou copolymères comportant comme constituant principal de la chaîne des motifs répondant aux formules (I) ou (II) :

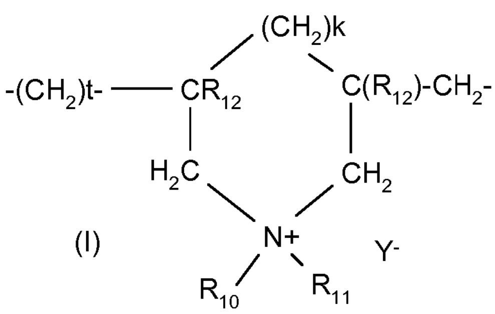
(I)

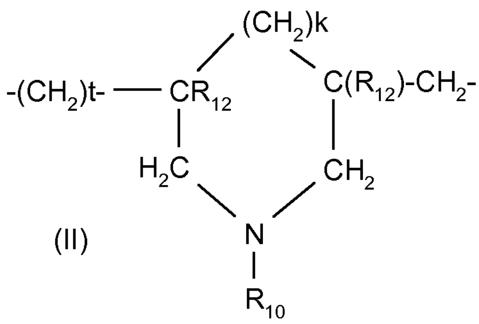
(II)

dans lesquelles

- k et t sont égaux à 0 ou 1, la somme k + t étant égale à 1 ;
- R12 désigne un atome d'hydrogène ou un radical méthyle ;
- R10 et R11, indépendamment l'un de l'autre, désignent un groupement alkyle en C1-C6, un groupement hydroxyalkyle en C1-C5, un groupement amidoalkyle en C1-C4; ou bien R10 et R11 peuvent désigner conjointement avec l'atome d'azote auquel ils sont rattachés, un groupement hétérocyclique tel que pipéridinyle ou morpholinyle; R10 et R11, indépendamment l'un de l'autre, désignent de préférence un groupement alkyle en C1-C4;
- $Y^-$ est un anion tel que bromure, chlorure, acétate, borate, citrate, tartrate, bisulfate, bisulfite, sulfate, phosphate.

On peut citer plus particulièrement l'homopolymère de sels (par exemple chlorure) de diméthyldiallylammonium par exemple vendu sous la dénomination "MERQUAT 100" par la société NALCO, De préférence, les polymères de la famille (1) sont choisis parmi les homopolymères de dialkyl diallyl ammonium.

(2) les polymères de diammonium quaternaire comprenant des motifs récurrents de formule :

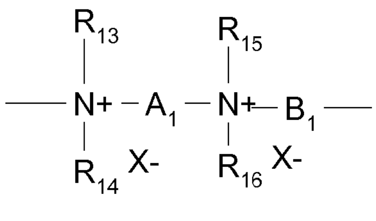
(III)

dans laquelle :

- R13, R14, R15 et R16, identiques ou différents, représentent des radicaux aliphatiques, alicycliques, ou arylaliphatiques comprenant de 1 à 20 atomes de carbone ou des radicaux hydroxyalkylaliphatiques en C1-C12, ou bien R13, R14, R15 et R16, ensemble ou séparément, constituent avec les atomes d'azote auxquels ils sont rattachés des hétérocycles comprenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
- A1 et B1 représentent des groupements divalents polyméthyléniques comprenant de 2 à 20 atomes de carbone, linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
- $X^-$ désigne un anion dérivé d'un acide minéral ou organique;

étant entendu que A1, R13 et R15 peuvent former avec les deux atomes d'azote auxquels ils sont rattachés un cycle pipérazinique ;
en outre si A1 désigne un radical alkylène ou hydroxyalkylène linéaire ou ramifié, saturé ou insaturé, B1 peut également désigner un groupement (CH2)n-CO-D-OC-(CH2)p-, avec n et p, identiques ou différents, étant des entiers variant de 2 à 20, et D désignant :

a) un reste de glycol de formule -O-Z-O-, où Z désigne un radical hydrocarboné linéaire ou ramifié ou un groupement répondant à l'une des formules suivantes: -(CH2CH2O)x-CH2CH2- et -[CH2CH(CH3)O]y-CH2CH(CH3)- où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine;
c) un reste de diamine bis-primaire de formule -NH-Y-NH- où Y désigne un radical hydrocarboné linéaire ou ramifié, ou bien le radical divalent -CH2-CH2-S-S-CH2-CH2- ;
d) un groupement uréylène de formule -NH-CO-NH- .

De préférence, $X^-$ est un anion tel que le chlorure ou le bromure. Ces polymères ont une masse molaire moyenne en nombre (Mn) généralement comprise entre 1000 et 100000.
On peut citer plus particulièrement les polymères cationiques qui sont constitués de motifs récurrents répondant à la formule :

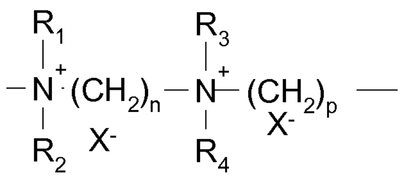
(IV)

dans laquelle R1, R2, R3 et R4, identiques ou différents, désignent un radical alkyle ou hydroxyalkyle ayant de 1 à 4 atomes de carbone, n et p sont des nombres entiers variant de 2 à 20, et X- est un anion dérivé d'un acide minéral ou organique.
Un composé de formule (IV) particulièrement préféré est celui pour lequel R1, R2, R3 et R4 représentent un radical méthyle, n=3, p=6 et X = Cl, dénommé Hexadimethrine chloride selon la nomenclature INCI (CTFA).
De préférence, le ou les polymère(s) cationique(s) est/sont choisi(s) parmi les homopolymères de dialkyl diallyl ammonium, en particulier les homopolymères de sels de diméthyldiallylammonium, les polymères constitués de motifs récurrents répondant à la formule (IV) ci-dessus, en particulier le poly(dimethyliminio)-1,3-propanediyl(dimethyliminio)-1,6-hexanediyldichloride, dont le nom INCI est hexadimethrine chloride ; et leurs mélanges.
Lorsqu'ils sont présents, la teneur totale en polymères cationiques (différents des polymères associatifs et des polymères fixants) dans la composition (A) peut varier de 0,01 à 10% en poids par rapport au poids de la composition, de préférence de 0,1 à 7 %, par rapport au poids de la composition, encore plus avantageusement de 0,5à 5 % en poids, mieux de 0,5 à 3 % en poids par rapport au poids de la composition (A).

***Tensioactifs***

De préférence, la composition (A) comprend un ou plusieurs agents tensioactifs, ceux-ci peuvent être choisis parmi les agents tensioactifs anioniques, les agents tensioactifs amphotères ou zwittérioniques, les agents tensioactifs

non-ioniques et les agents tensioactifs cationiques, et leurs mélanges, de préférence parmi les agents tensioactifs non ioniques, les agents tensioactifs cationiques et leurs mélanges.

Les tensioactifs non ioniques utilisables selon l'invention peuvent être choisis parmi :

- les alcools, les alpha-diols et les alkyl($C_1$-$C_{20}$)phénols, ces composés étant polyéthoxylés et/ou polypropoxylés et/ou polyglycérolés, le nombre de groupements oxyde d'éthylène et/ou oxyde de propylène pouvant aller de 1 à 100, et le nombre de groupements glycérol pouvant aller de 2 à 30; ou bien ces composés comprenant au moins une chaîne grasse comportant de 8 à 40 atomes de carbone, notamment de 16 à 30 atomes de carbone ; en particulier les alcools comprenant au moins une chaîne alkyle en $C_8$ à $C_{40}$, saturés ou non, linéaires ou ramifiés, oxyéthylénés comprenant de 1 à 100 moles d'oxyde d'éthylène, de préférence de 2 à 50, plus particulièrement de 2 à 40 moles d'oxyde d'éthylène et comportant une ou deux chaînes grasses;
- les condensats d'oxyde d'éthylène et d'oxyde de propylène sur des alcools gras;
- les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4;
- les esters d'acides gras du sorbitane éthoxylés ayant de préférence de 2 à 40 motifs d'oxyde d'éthylène ;
- les esters d'acides gras du saccharose ;
- les esters d'acides gras polyoxyalkylénés, de préférence polyoxyéthylénés ayant de 2 à 150 moles d'oxyde d'éthylène dont les huiles végétales oxyéthylénées ;
- les dérivés de N-(alkyl en $C_6$-$C_{24}$)glucamine,
- les oxydes d'amines tels que les oxydes d'(alkyl en $C_{10}$-$C_{14}$)amines ou les oxydes de N-(acyl en $C_{10}$-$C_{14}$)-aminopropylmorpholine ;
- et leurs mélanges.

On peut encore citer les tensioactifs non ioniques de type alkyl(poly)glycoside,

Parmi les produits commerciaux, on peut citer les produits vendus par la société COGNIS sous les dénominations PLANTAREN® (600 CS/U, 1200 et 2000) ou PLANTACARE® (818, 1200 et 2000); les produits vendus par la société SEPPIC sous les dénominations ORAMIX CG 110 et ORAMIX® NS 10; les produits vendus par la société BASF sous la dénomination LUTENSOL GD 70 ou encore les produits vendus par la société CHEM Y sous la dénomination AG10 LK.

De préférence, on utilise les alkyl $C_8$/$C_{16}$-(poly)glycoside 1,4, notamment en solution aqueuse à 53%, tels que ceux commercialisés par COGNIS sous la référence PLANTACARE® 818 UP.

Préférentiellement, les tensioactifs non ioniques sont choisis parmi :

- les alcools gras oxyéthylénés, comportant au moins une chaîne alkyle en $C_8$ à $C_{40}$, notamment en $C_8$-$C_{20}$, encore mieux en $C_{10}$-$C_{18}$ , saturée ou non, linéaire ou ramifiée, et comprenant de 1 à 100 moles d'oxyde d'éthylène, de préférence de 2 à 50, plus particulièrement de 2 à 40 moles, voire de 3 à 20 moles d'oxyde d'éthylène;, et
- les (alkyl $C_6$-$C_{24}$)(poly)glycosides, et plus particulièrement les (alkyl $C_8$-$C_{18}$)(poly)glycosides ;
- et leurs mélanges ;

et plus préférentiellement encore parmi les (alkyl $C_6$-$C_{24}$)(poly)glycosides, préférentiellement les (alkyl $C_8$-$C_{18}$)(poly)glycosides.

Selon un premier mode de réalisation, le ou les agents tensioactifs sont non ioniques, de préférence choisis parmi les (alkyl en $C_6$-$C_{24}$)polyglycosides.

Selon un mode de réalisation préféré, la composition (A) comprend au moins un ou plusieurs agents tensioactifs cationiques. De préférence, le ou les tensioactifs cationiques sont choisis parmi les sels d'amines grasses primaires, secondaires ou tertiaires, éventuellement polyoxyalkylénées, les sels d'ammonium quaternaire, et leurs mélanges.

On peut également utiliser le chlorure de béhénoylhydroxypropyltriméthylammonium, par exemple, proposé par la société KAO sous la dénomination Quartamin BTC 131.

De préférence, les sels d'ammonium contenant au moins une fonction ester contiennent deux fonctions esters.

De préférence, le ou les agents tensioactifs cationiques, sont choisis parmi les sels de cétyltriméthylammonium, de béhényltriméthylammonium, de dipalmitoyléthylhydroxy éthylméthylammonium, et leurs mélanges, et plus particulièrement le chlorure de béhényltriméthylammonium, le chlorure de cétyltriméthylammonium, le méthosulfate de dipalmitoyléthylhydroxy éthylammonium, et leurs mélanges.

La composition (A) comprend, de préférence un ou plusieurs agents tensioactifs en une teneur totale allant de 0,01 à 20 % en poids, plus préférentiellement de 0,05 à 10 % en poids, mieux de 0,1 à 5 % en poids, par rapport au poids total de la composition (A).

La composition (A) comprend, de préférence un ou plusieurs agents tensioactifs non ioniques en une teneur totale allant de 0,01 à 10 % en poids, plus préférentiellement de 0,05 à 5 % en poids, mieux de 0,1 à 3 % en poids, par

rapport au poids total de la composition (A).

La composition (A) comprend de préférence un ou plusieurs agents tensioactifs cationiques en une teneur totale allant de 0,01 à 10 % en poids, plus préférentiellement de 0,05 à 5 % en poids, mieux de 0,1 à3 % en poids, par rapport au poids total de la composition (A).

De manière préférée, le ou les agents tensioactifs sont choisis parmi les tensioactifs cationiques ou non ioniques, et leurs mélanges, préférentiellement cationiques. De préférence la composition (A) comprend au moins un ou plusieurs tensioactifs cationiques et un ou plusieurs tensioactifs non ioniques.

**Composition Oxydante (B)**

**Agents oxydants** :

La composition oxydante (B) mise en oeuvre selon l'invention contient un ou plusieurs agent(s) oxydant(s) chimique(s), de préférence choisi(s) parmi le peroxyde d'hydrogène et/ou un ou plusieurs système(s) générateur(s) de peroxyde d'hydrogène.

Par « agent oxydant chimique » on entend un agent oxydant différent de l'oxygène de l'air.

De préférence, le ou les agents oxydants chimiques sont choisis parmi le peroxyde d'hydrogène, les sels peroxygénés comme par exemple les persulfates, les perborates, les peracides et leurs précurseurs, les percarbonates de métaux alcalins ou alcalino-terreux tel que le peroxyde de carbonate de sodium ou appelé percarbonate de sodium et les peracides et leurs précurseurs ; les bromates ou ferricyanures de métaux alcalins, les agents oxydants chimiques solides générateurs de peroxyde d'hydrogène tels que le peroxyde d'urée et les complexes polymériques pouvant libérer du peroxyde d'hydrogène notamment ceux comprenant un monomère hétérocyclique vinylique tels que les complexes polyvinylpyrrolidone/$H_2O_2$ en particulier se présentant sous forme de poudres ; les oxydases produisant du peroxyde d'hydrogène en présence d'un substrat adéquat (par exemple le glucose dans le cas de glucose oxydase ou l'acide urique avec l'uricase).

De manière préférée, le ou les agents oxydants chimiques sont choisis parmi le peroxyde d'hydrogène, le peroxyde d'urée, les bromates ou ferricyanures de métaux alcalins, les sels peroxygénés, et les mélanges de ces composés,

De manière particulièrement préférée, l'agent oxydant chimique est du peroxyde d'hydrogène.

De manière préférée, le ou les agents oxydants chimiques représentent de 0,05 à 40 % en poids, de préférence de 0,5 à 30 % en poids, plus préférentiellement de 1 à 20 % en poids, et mieux de 1,5 à 15 % en poids par rapport au poids total de la composition oxydante (B).

De préférence, la composition oxydante (B)selon l'invention ne contient pas de sels peroxygénés.

Comme indiqué précédemment, la composition oxydante (B) peut comprendre une ou plusieurs gommes de scléroglucane, de préférence en une teneur totale supérieure ou égale à 0,5% en poids par rapport au poids de la composition.

De préférence, lorsque la composition (B) comprend une ou plusieurs gommes de scléroglucane, leur teneur totale représente de préférence de 0,5 à 10 % en poids, plus préférentiellement de 0,5 à 5 % en poids, et encore plus préférentiellement de 0,5 à 3 % en poids, voire de 0,7 à 2% en poids, par rapport au poids total de la composition oxydante (B).

Selon un mode de réalisation préféré de l'invention, la composition oxydante (B) comprend une ou plusieurs gommes de scléroglucane, de préférence en une teneur totale supérieure ou égale à 0,5% en poids,

Plus préférentiellement, la composition oxydante (B) comprend une ou plusieurs gommes de scléroglucane en une teneur totale supérieure ou égale à 0,5% en poids, de préférence de 0,5 à 10 % en poids, plus préférentiellement de 0,5 à 5 % en poids, et encore plus préférentiellement de 0,5 à 3 % en poids, voire de 0,7 à 2% en poids, par rapport au poids total de la composition oxydante (B)

La composition oxydante (B) peut également renfermer divers composés additionnels ou divers adjuvants utilisés classiquement dans les compositions pour la coloration des cheveux notamment tels que définis précédemment, en particulier tels que un ou plusieurs agents tensioactifs tels que décrits précédemment.

Cette composition oxydante (B) peut également comprendre un ou plusieurs solvants organiques hydrosolubles tels que décrits ci-après..

Enfin, la composition oxydante (B) se présente sous diverses formes, comme par exemple une solution, une émulsion ou un gel.

*Milieu*

Les compositions mises en oeuvre selon l'invention sont cosmétiquement acceptable et comprennent en conséquence un milieu cosmétiquement acceptable.

Par « milieu cosmétiquement acceptable », on entend un milieu compatible avec les fibres kératiniques.

Le milieu cosmétiquement acceptable approprié pour la coloration des fibres kératiniques, appelé aussi « support » de coloration, comprend généralement de l'eau ou un mélange d'eau et d'au moins un solvant organique pour solubiliser les composés qui ne seraient pas suffisamment solubles dans l'eau.

Plus particulièrement, les solvants organiques sont choisis parmi les monoalcools ou les diols, linéaires ou ramifiés, de préférence saturés, comprenant de 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; le glycérol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en $C_1$-$C_4$, comme par exemple, le monoéthyléther ou le monobutyléther du diéthylèneglycol, seuls ou en mélange.

Les solvants usuels décrits ci-dessus, s'ils sont présents, représentent habituellement de 1 à 40% en poids, plus préférentiellement de 5 à 30% en poids, par rapport au poids total de la composition.

Les compositions mises en oeuvre selon l'invention comprennent généralement de l'eau ou un mélange d'eau et d'un ou plusieurs solvants organiques ou un mélange de solvants organiques.

La composition colorante (A) et la composition oxydante (B) selon l'invention comprennent de préférence de l'eau.

De préférence la teneur en eau varie de 5 à 95% en poids, plus préférentiellement de 10 à 90 % en poids, mieux de 20 à 80 % en poids, par rapport au poids total de la composition (A)

La composition oxydante (B) est généralement une composition aqueuse. Par composition aqueuse, on entend au sens de l'invention une composition comprenant plus de 20 % en poids d'eau, de préférence plus de 30 % en poids d'eau, et de manière encore plus avantageuse plus de 40 % en poids d'eau.

La composition oxydante (B) est généralement une composition aqueuse. La composition oxydante (B) comprend usuellement de l'eau qui représentent généralement de 10 à 98 % en poids, de préférence de 20 à 96 % en poids, de préférence de 50 à 95 % en poids, par rapport au poids total de la composition.

***pH du milieu***

Le pH de la composition (A) mise en oeuvre dans le procédé selon l'invention varie généralement de 1 à 12. De façon préférée, le pH de la composition (A) selon l'invention est basique.

Par « pH basique » au sens de la présente invention, on entend un pH supérieur à 7.

De préférence, le pH de la composition (A) selon l'invention est supérieur à 8, et particulièrement va de 8,5 à 12.

De préférence le pH de la composition est compris entre 9 et 12. Habituellement, le pH de la composition (B), est inférieur à 7. Le pH de la composition (B) de l'invention est avantageusement compris entre 1 et 7, de préférence entre 1 et 4, et plus préférentiellement de 1,5 à 3,5.

*Ajusteur de pH*

Le milieu cosmétiquement acceptable peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux comme par exemple l'acide chlorhydrique, (ortho)phosphorique, l'acide borique, l'acide nitrique, l'acide sulfurique ou les acides organiques comme par exemple les composés comprenant au moins une fonction acide sulfonique, une fonction acide phosphonique ou une fonction acide phosphorique, ou les composés à fonction acide carboxylique tels que ceux décrits précédemment.

***Autres additifs***

Les compositions mises en oeuvre dans le procédé selon l'invention peut également renfermer divers additifs utilisés classiquement dans les compositions pour la coloration des cheveux, tels que des agents épaississants minéraux, et en particulier des charges telles que des argiles, le talc ; des agens épaississants organiques différents des gommes de scléroglucane ;des agents antioxydants ; des agents de pénétration ; des agents séquestrants ; des parfums ; des agents dispersants ; des agents filmogènes ; des céramides ; des agents conservateurs ; des agents opacifiants, des corps gras et/ou des colorants directs additionnels.

Les additifs ci-dessus sont en général présents en quantité comprise pour chacun d'eux entre 0,01 et 40 % en poids par rapport au poids de la composition, de préférence entre 0,1 et 20 % en poids par rapport au poids de la composition (A) et/ou de la composition (B).

Bien entendu, l'homme de l'art veillera à choisir ce ou ces éventuels composés complémentaires de manière telle que

les propriétés avantageuses attachées intrinsèquement à la composition ou aux composition(s) utiles dans le procédé de coloration conforme à l'invention ne soient pas, ou substantiellement pas, altérées par la ou les adjonctions envisagées.

**Dispositif de distribution**

Le dispositif de distribution selon l'invention est constitué d'un récipient comprenant:

i) au moins deux compartiments (a) et (b) séparés l'un de l'autre, le compartiment (a) comprenant une composition (A) comprenant :

- un ou plusieurs colorant(s) d'oxydation ;
- un ou plusieurs agents alcalins ; et
  le compartiment (b) comprenant une composition oxydante (B) comprenant ;
- un ou plusieurs agent(s) oxydants chimiques, de préférence choisi parmi le peroxyde d'hydrogène et/ou un ou plusieurs système(s) générateur(s) de peroxyde d'hydrogène, de préférence parmi le peroxyde d'hydrogène ; et
- l'une et/ou l'autre des compositions (A) et/ou (B) comprenant une ou plusieurs gommes de scléroglucane, de préférence en une teneur totale en poids supérieure ou égale à 0,5% par rapport au poids total de la(des) composition(s) (A) et/ou (B) la(les) contenant

ii)un moyen de distribution muni d'au moins un orifice de distribution, en communication avec les compartiments (a) et (b), permettant de délivrer de manière simultanée les compositions A et B, à l'état mélangé ou séparé.

Selon un mode de réalisation particulier, le dispositif de distribution selon l'invention est constitué d'un récipient comprenant:

i) au moins deux compartiments (a) et (b) séparés l'un de l'autre, le compartiment (a) comprenant une composition (A) comprenant :

- un ou plusieurs colorant(s) d'oxydation ;
- un ou plusieurs agents alcalins ; et
  le compartiment (b) comprenant une composition oxydante (B) comprenant ;
- un ou plusieurs agent(s) oxydants chimiques, de préférence choisi parmi le peroxyde d'hydrogène et/ou un ou plusieurs système(s) générateur(s) de peroxyde d'hydrogène, de préférence parmi le peroxyde d'hydrogène ; et
- l'une et/ou l'autre des compositions (A) et/ou (B) comprenant une ou plusieurs gommes de scléroglucane en une teneur totale en poids supérieure ou égale à 0,5% par rapport au poids total de la(des) composition(s) (A) et/ou (B) la(les) contenant

ii)un moyen de distribution muni d'au moins un orifice de distribution, en communication avec les compartiments (a) et (b), permettant de délivrer de manière simultanée les compositions A et B, à l'état mélangé ou séparé.

Selon un mode de réalisation préféré de l'invention, le dispositif de distribution selon l'invention est constitué d'un récipient comprenant:

i) au moins deux compartiments (a) et (b) séparés l'un de l'autre, le compartiment (a) comprenant une composition (A) comprenant :

- un ou plusieurs colorant(s) d'oxydation ;
- une ou plusieurs gomme(s) de scléroglucane, de préférence en une totale supérieure ou égale à 0,5% en poids par rapport au poids totale de la composition (A)
- un ou plusieurs agents alcalins ; et
  le compartiment (b) comprenant une composition oxydante (B) comprenant ;
- un ou plusieurs agent(s) oxydants chimiques, de préférence choisi parmi le peroxyde d'hydrogène et/ou un ou plusieurs système(s) générateur(s) de peroxyde d'hydrogène, de préférence parmi le peroxyde d'hydrogène ; et
- une ou plusieurs gommes de scléroglucane, de préférence en une teneur totale en poids supérieure ou égale à 0,5% par rapport au poids total de la composition (B)

ii)un moyen de distribution muni d'au moins un orifice de distribution, en communication avec les compartiments (a) et (b), permettant de délivrer de manière simultanée les compositions A et B, à l'état mélangé ou séparé.

Selon un mode de réalisation encore plus préférentiel, le dispositif de distribution selon l'invention est constitué d'un récipient comprenant:

i) au moins deux compartiments (a) et (b) séparés l'un de l'autre, le compartiment (a) comprenant une composition (A) comprenant :

- un ou plusieurs colorant(s) d'oxydation ;
- une ou plusieurs gomme(s) de scléroglucane en une totale supérieure ou égale à 0,5% en poids par rapport au poids totale de la composition (A)
- un ou plusieurs agents alcalins ; et
  le compartiment (b) comprenant une composition oxydante (B) comprenant ;
- un ou plusieurs agent(s) oxydants chimiques, de préférence choisi parmi le peroxyde d'hydrogène et/ou un ou plusieurs système(s) générateur(s) de peroxyde d'hydrogène, de préférence parmi le peroxyde d'hydrogène ; et
- une ou plusieurs gommes de scléroglucane, de préférence en une teneur totale en poids supérieure ou égale à 0,5% par rapport au poids total de la composition (B)

ii)un moyen de distribution muni d'au moins un orifice de distribution, en communication avec les compartiments (a) et (b), permettant de délivrer de manière simultanée les compositions A et B, à l'état mélangé ou séparé.

De préférence le récipient du dispositif est pressurisé (dispositif aérosol). En d'autres termes, il comprend un ou plusieurs gaz propulseurs.

A titre de gaz propulseur convenant à la mise en oeuvre de l'invention, on peut citer les gaz habituellement employés dans le domaine de la cosmétique, en particulier les hydrocarbures volatiles, éventuellement halogénés, par exemples le n-butane, le propane, l'isobutane, le pentane et leurs dérivés halogénés ; le dioxyde de carbone, l'oxyde nitreux, le diméthyléther, l'azote, l'oxygène, seuls ou en mélanges.

Les parois du récipient contenant les compartiments (a) et (b) sont de préférence rigides, le récipient pouvant être dans ce cas un bidon, par exemple en matériau métallique ou plastique.

Selon un mode de réalisation préféré, les compartiments (a) et (b) sont des poches flexibles. Elles peuvent être en matériau métallique, tel que l'aluminium, ou plastique.

Selon cette configuration, le gaz propulseur se trouve dans le volume défini entre les parois du récipient et les poches flexibles.

Le dispositif comprend un moyen de distribution muni d'au moins un orifice de distribution, en communication avec les compartiments (a) et (b), permettant de délivrer de manière simultanée les compositions (A) et (B), sous forme séparée (cote à cote par exemple) ou sous forme de mélange, par l'intermédiaire d'au moins un orifice de distribution.

De préférence, le moyen permettant de délivrer les compositions comprend au moins une valve de distribution. Selon un mode de réalisation, chaque compartiment est surmonté d'une valve.

Selon un mode de réalisation, les compartiments sont surmontés d'une valve unique les reliant.

La ou les valves sont en communication fluidique sélective avec l'intérieur du ou des compartiments via un orifice d'entrée de la valve, la communication étant établie en réponse à l'actionnement d'un moyen d'actionnement, tel qu'un bouton-poussoir.

Le moyen de distribution du dispositif peut comprendre un diffuseur qui coiffe la ou les valves.

Selon une variante préférée, le dispositif comprend un diffuseur unique qui coiffe les deux valves. Le bouton-poussoir peut faire partie du diffuseur.

Le diffuseur peut être muni d'un ou plusieurs conduits de distribution prévu(s) pour acheminer la ou les compositions jusqu'à un ou plusieurs orifices de distribution.

Lorsque le dispositif comprend un unique diffuseur, il peut être muni de deux conduits d'acheminement des compositions, chaque conduit communiquant avec l'orifice de sortie d'une valve.

Selon un premier mode de réalisation, les deux conduits aboutissent chacun à un orifice de distribution (ne communiquant pas entre eux avant l'orifice de distribution). Selon cette configuration, le mélange des compositions n'est réalisé qu'après avoir été distribué (donc après les orifices de distribution).

Selon un deuxième mode de réalisation, les deux conduits aboutissent dans une chambre de mélange équipée d'un mélangeur statique, de laquelle un seul conduit est dirigé vers un unique orifice de distribution. Selon cette configuration, le mélange des compositions est réalisé juste avant son expulsion du dispositif.

Ainsi, les compositions (A) et (B) peuvent être délivrées sous forme de mélange préalablement à l'application sur les fibres kératiniques, ou être délivrées simultanément sous forme séparée, le mélange de faisant après application sur les fibres kératiniques (lorsque les compositions sortent simultanément côte à côte).

De préférence, selon ce mode de réalisation, les compartiments (a) et (b) sont des poches flexibles, une valve de distribution surmontant chacun des compartiments, un unique diffuseur coiffant les deux valves.

Il est à noter que la ou les valves de distribution, de même que la teneur en gaz propulseur(s), sont adaptées de manière à permettre la distribution des compositions dans des proportions respectives appropriées.
Dans la pratique le rapport pondéral composition (A)/composition (B) distribué varie de 0,1 à 10 de préférence de 0,2 à 2, mieux de 0,3 à 1.

**Procédé de coloration**

L'invention a également pour objet un procédé de coloration des fibres kératiniques, de préférence humaines, mettant en oeuvre le dispositif précédemment décrit.
Selon un mode de réalisation particulier, l'invention a également pour objet un procédé de coloration des fibres kératiniques, de préférence humaines, mettant en oeuvre le dispositif précédemment décrit.
Plus précisément, l'invention a pour objet un procédé de coloration des fibres kératiniques, de préférence des fibres kératiniques humaines telles que les cheveux, mettant en oeuvre l'application sur les fibres:

a) d'une composition colorante (A) comprenant :

- un ou plusieurs colorant(s) d'oxydation ;
- ;
- un ou plusieurs agents alcalins ; et

b) d'une composition oxydante (B) comprenant :

- un ou plusieurs agent(s) oxydants chimiques, de préférence choisi parmi le peroxyde d'hydrogène et/ou un ou plusieurs système(s) générateur(s) de peroxyde d'hydrogène, de préférence parmi le peroxyde d'hydrogène ; et
- l'une et/ou l'autre des compositions (A) et/ou (B) comprenant une ou plusieurs gommes de scléroglucane en une teneur totale en poids supérieure ou égale à 0,5% par rapport au poids total de la (des) composition(s) (A) et/ou (B) la (les) contenant ;

les compositions (A) et (B) étant conditionnées dans un dispositif constitué d'un récipient, de préférence pressurisé, comprenant au moins deux compartiments (a) et (b) séparés l'un de l'autre, le compartiment (a) comprenant la composition (A) et le compartiment (b) comprenant la composition (B), le dispositif comprenant un moyen de distribution muni d'au moins un orifice de distribution, en communication avec les compartiments (a) et (b), permettant de délivrer les compositions (A) et (B) de manière simultanée.

**Selon un mode de réalisation préféré,** l'invention a pour objet un procédé de coloration des fibres kératiniques, de préférence des fibres kératiniques humaines telles que les cheveux, mettant en oeuvre l'application sur les fibres:

a) d'une composition colorante (A) comprenant :

- un ou plusieurs colorant(s) d'oxydation ;
- une ou plusieurs gomme(s) de scléroglucane, de préférence en une totale supérieure ou égale à 0,5% en poids par rapport au poids totale de la composition (A) ;
- un ou plusieurs agents alcalins ; et

b) une composition oxydante (B) comprenant :

- un ou plusieurs agent(s) oxydants chimiques, de préférence choisi parmi le peroxyde d'hydrogène et/ou un ou plusieurs système(s) générateur(s) de peroxyde d'hydrogène, de préférence parmi le peroxyde d'hydrogène ; et
- une ou plusieurs gommes de scléroglucane, de préférence en une teneur totale en poids supérieure ou égale à 0,5% par rapport au poids total de la (B);

les compositions (A) et (B) étant conditionnées dans un dispositif constitué d'un récipient, de préférence pressurisé, comprenant au moins deux compartiments (a) et (b) séparés l'un de l'autre, le compartiment (a) comprenant la composition (A) et le compartiment (b) comprenant la composition (B), le dispositif comprenant un moyen de distribution muni d'au moins un orifice de distribution, en communication avec les compartiments (a) et (b), permettant de délivrer les compositions (A) et (B) de manière simultanée.

Plus préférentiellement, l'invention a pour objet un procédé de coloration des fibres kératiniques, de préférence

des fibres kératiniques humaines telles que les cheveux, mettant en oeuvre l'application sur les fibres:

a) une composition colorante (A) comprenant :

- un ou plusieurs colorant(s) d'oxydation ;
- une ou plusieurs gomme(s) de scléroglucane en une totale supérieure ou égale à 0,5% en poids par rapport au poids totale de la composition (A) ;
- un ou plusieurs agents alcalins ; et

b) une composition oxydante (B) comprenant :

- un ou plusieurs agent(s) oxydants chimiques, de préférence choisi parmi le peroxyde d'hydrogène et/ou un ou plusieurs système(s) générateur(s) de peroxyde d'hydrogène, de préférence parmi le peroxyde d'hydrogène ; et
- une ou plusieurs gommes de scléroglucane, de préférence en une teneur totale en poids supérieure ou égale à 0,5% par rapport au poids total de la (B);

les compositions (A) et (B) étant conditionnées dans un dispositif constitué d'un récipient, de préférence pressurisé, comprenant au moins deux compartiments (a) et (b) séparés l'un de l'autre, le compartiment (a) comprenant la composition (A) et le compartiment (b) comprenant la composition (B), le dispositif comprenant un moyen de distribution muni d'au moins un orifice de distribution, en communication avec les compartiments (a) et (b), permettant de délivrer les compositions (A) et (B) de manière simultanée.

Les compositions (A) et (B) délivrées au moyen du dispositif selon l'invention sont éventuellement mélangées par malaxage sur les fibres kératiniques (en particulier lorsque les compositions (A) et (B) sont délivrées côte à côte), et laissées en place pour une durée, en général, de l'ordre de 1 minute à 1 heure, de préférence de 5 minutes à 30 minutes.
La température durant le temps de pose est classiquement comprise entre la température ambiante (entre 15 à 25°C) et 80°C, de préférence entre la température ambiante et 60°C.
A l'issue du traitement, les matières kératiniques sont éventuellement rincées à l'eau, subissent éventuellement un lavage suivi d'un rinçage à l'eau, avant d'être séchées ou laissées à sécher.
De préférences les fibres kératiniques sont des fibres kératiniques humaines, de préférence des cheveux humains.
L'invention a également pour objet une composition de coloration prête à l'emploi des fibres kératiniques, en particulier des fibres kératiniques humaines, telles que les cheveux, obtenue par mélange extemporané, au moment de l'emploi d'une composition (A) telle que définie précédemment;
et d'une composition (B) telle que définie précédemment.
Selon un mode particulier de l'invention, le ou les agents oxydants chimiques représente(nt) de préférence une teneur totale variant de 0,1 à 20 % en poids, de préférence de 0,5 à 15% en poids, ou encore plus préférentiellement de 1 à 10% en poids, par rapport au poids total de la composition prête à l'emploi (mélange des compositions A et B distribué).
Les exemples suivants servent à illustrer l'invention sans toutefois présenter un caractère limitatif.
En particulier, la coloration des fibres kératiniques obtenue dans ces exemples peut être avantageusement évaluée dans le système CIE L* a* b*, au moyen d'un Spectrocolorimètre Datacolor Spectraflash SF600X.
Dans ce système L* a* b*, les trois paramètres désignent respectivement l'intensité de la couleur (L*), a* indique l'axe de couleur vert/rouge et b* l'axe de couleur bleu/jaune. Plus la valeur de L* est élevée, plus la couleur est claire. Plus la valeur de a* est élevée, plus la couleur est rouge, plus la valeur de b* est élevée, plus la couleur est jaune.
La variation (ou l'importance) de la coloration entre les mèches de cheveux non traitées et les mèches de cheveux après traitement est définie par le paramètre DE* et est calculé selon l'équation suivante :

$$DE^* = \sqrt{(L^* - L_o^*)^2 + (a^* - a_o^*)^2 + (b^* - b_o^*)^2} \quad \text{(i)}$$

Dans cette équation, les paramètres L*, a* et b* représentent les valeurs mesurées sur les mèches de cheveux après la coloration et les paramètres $L_0$*, $a_0$* et $b_0$* représentent les valeurs mesurées sur les mèches de cheveux non traitées. Plus la valeur de DE* est grande, meilleure est la coloration des fibres kératiniques.
Dans le système CIE L*, a*, b*, la chromaticité est calculée selon l'équation suivante:

$$C^* = \sqrt{a^{*2} + b^{*2}}$$

Plus la valeur de C* est élevée et plus la coloration est chromatique.

Exemple 1:

On a préparé la composition colorante suivante à partir des ingrédients suivants dans les proportions suivantes indiquées en gramme :

| | Composition A2 selon l'invention |
|---|---|
| ETHANOLAMINE | 3,23 |
| EDTA | 0,2 |
| SODIUM SULFITE | 0,5 |
| TOLUENE-2,5-DIAMINE | 1,03 |
| 2,4-DIAMINOPHENOXYETHANOL HCl | 0,021 |
| 2-METHYLRESORCINOL | 0,27 |
| N,N-BIS(2-HYDROXYETHYL)-p-PHENYLENEDIAMINE SULFATE | 0,13 |
| RESORCINOL | 0,28 |
| m-AMINOPHENOL | 0,45 |
| FRAGRANCE | qs |
| CETYL HYDROXYETHYLCELLULOSE | 0,2 |
| SCLEROTIUM GUM | 1 |
| EAU | qs 100 |
| GLYCERIN | 10 |
| COCO-BETAINE | 0,15 |
| CAPRYLYL/CAPRYL GLUCOSIDE | 0,6 |
| ASCORBIC ACID | 0,4 |

**Evaluation visuelle de la stabilité de la composition A2**

La stabilité des compositions colorantes a été évaluée en observant les compositions à T0 (immédiatement après préparation de la composition) puis après 2 mois de stockage à température ambiante (25°C), et après 2 mois de stockage à 45°C.

| | Composition A2 selon l'invention |
|---|---|
| Observation à T0 (immédiatement après préparation) | Homogène (Pas de Déphasage)<br>Texture : Gel lisse |
| Observation après 2 mois à 25°C | Homogène (Pas de Déphasage)<br>Texture : Gel lisse |
| Observation après 2 mois à 45°C | Homogène (Pas de Déphasage)<br>Texture : Gel lisse |

On observe que la composition A2 du procédé selon l'invention sont homogènes et forment un gel translucide à T0. Après 2 mois à température ambiante ou à 45° C.

On a préparé la composition oxydante B3 suivante à partir des ingrédients suivants dans les proportions

suivantes indiquées en gramme.

| | Composition oxydante B3 selon l'invention |
|---|---|
| HYDROGEN PEROXIDE | 6 |
| PHOSPHORIC ACID | Qs pH 2,2 ± 0,2 |
| TETRASODIUM ETIDRONATE | 0,2 |
| TETRASODIUM PYROPHOSPHATE | 0,04 |
| SODIUM SALICYLATE | 0,035 |
| SCLEROTIUM GUM | 1,8 |
| EAU | Qs 100 |

On observe que la composition B3 du procédé selon l'invention est homogène et forme un gel translucide à T0, après 2 mois à température ambiante ou à 45° C.
Les compositions A2 et B3 sont conditionnées dans un récipient aérosol comprenant deux poches flexibles muni d'un diffuseur comprenant un bouton poussoir et sont distribuées simultanément côte à côte dans les proportions 1/1 et sont appliquées sur des mèches de cheveux naturels à 90% blancs.
Le rapport de bain « mélange/mèche » est respectivement de 10/1 (g/g). Les compositions se mélangent rapidement sur les cheveux : le mélange est translucide et se répartit aisément de façon homogène sur les cheveux.
Les qualités d'usage sont bonnes : bon caractère mouillant/glissant, bonne facilité d'application, bonne adhérence racine, bonne consistance sur la tête, bonne facilité d'allongement le long des mèches de fibres.
Le temps de pause de 30 minutes, sur plaque chauffante réglée à 27°C. A l'issue du temps de pause, les mèches sont rincées puis séchées sous casque à 40°C.
Les cheveux sont ensuite facilement rincés, puis lavés avec un shampooing standard et séchés.
La couleur des mèches a été évaluée dans le système CIE L*a*b* au moyen d'un spectrocolorimètre Datacolor Spectraflash SF600X.

| | L* |
|---|---|
| Mélange composition A2 et B3 | 21,48 |

On obtient une coloration intense des fibres kératiniques.

## Revendications

**1.** Dispositif de distribution d'un produit de coloration des fibres kératiniques constitué d'un récipient comprenant:

i) au moins deux compartiments (a) et (b) séparés l'un de l'autre,

a) le compartiment a comprenant une composition (A) comprenant :

- -un ou plusieurs colorant(s) d'oxydation ;
- -un ou plusieurs agents alcalins ; dans lequel le ou les agents alcalins est/sont choisis parmi l'ammoniaque, les métasilicates de métaux alcalins ou alcalino terreux les alcanolamines, les acides aminés, et leurs mélanges et

b) le compartiment b comprenant une composition oxydante B comprenant ;

- un ou plusieurs agent(s) oxydants chimiques, de préférence choisi(s) parmi le peroxyde d'hydrogène et/ou un ou plusieurs système(s) générateur(s) de peroxyde d'hydrogène, ; et

- l'une et/ou l'autre des compositions (A) et/ou (B) comprenant une ou plusieurs gommes de scléroglucane,;

ii) un moyen de distribution muni d'au moins un orifice de distribution, en communication avec les compartiments (a) et (b), permettant de délivrer de manière simultanée les compositions A et B, à l'état mélangé ou séparé.

2. Dispositif selon la revendication précédente **caractérisé en ce que** la composition (A) comprend une ou plusieurs gommes de scléroglucane, de préférence en une teneur totale en poids supérieure ou égale à 0,5% par rapport au poids total de la composition (A), et la composition (B) comprend une ou plusieurs gommes de scléroglucane, de préférence en une teneur totale en poids supérieure ou égale à 0,5% par rapport au poids total de la composition (B).

3. Dispositif l'une quelconque des revendications précédentes, **caractérisé en ce que** la ou les gommes de scléroglucane représentent de 0,5 à 10 % en poids, plus préférentiellement de 0,5 à 5 % en poids, et encore plus préférentiellement de 0,5 à 3 % en poids, mieux de 0.5% à 2% en poids et encore plus préférentiellement de 0,7 à 1,5 % en poids, par rapport au poids total de la composition (A).

4. Dispositif selon l'une quelconque des revendications précédentes, **caractérisé en ce que** le ou les colorants d'oxydation sont choisi(s) parmi les bases d'oxydation benzéniques de préférence choisies parmi les paraphénylènediamines, les bis-phénylalkylènediamines, les para-aminophénols, les ortho-aminophénols, les bases hétérocycliques et leurs sels d'addition ; éventuellement combinées à un ou plusieurs coupleurs de préférence choisis parmi les méta-phénylènediamines, les méta-aminophénols, les méta-diphénols, les coupleurs naphthaléniques, et les coupleurs hétérocycliques ainsi que leurs sels d'addition.

5. Dispositif selon l'une quelconque des revendications précédentes, **caractérisé en ce que** le ou les agents alcalins sont choisis parmi les agents alcalins minéraux et/ou organiques, de préférence parmi un mélange d'au moins un agent alcalin minéral et d'au moins un agent alcalin organique ;

6. Dispositif selon l'une quelconque des revendications précédentes, **caractérisée** en que la composition comprend de l'ammoniaque (hydroxyde d'ammonium) et une ou plusieurs alcanolamine(s), de préférence la monoethanolamine.

7. Dispositif selon l'une quelconque des revendications précédentes, **caractérisé en ce que** la composition colorante (A) comprend un ou plusieurs agents tensioactifs, de préférence choisis parmi les tensioactifs cationiques et/ou non ioniques, le ou lesdits tensioactifs est/sont de préférence présents en une teneur totale allant de 0,01 à 20% , plus préférentiellement de 0,05 à 10% en poids, mieux de 0,1 à 5% en poids par rapport au poids totale de la composition colorante (A).

8. Dispositif selon l'une quelconque des revendications précédentes, **caractérisé en ce que** la composition colorante (A) comprend un ou plusieurs polymère(s) associatif(s), de préférence non ioniques(s) choisis parmi les celluloses modifiées par des groupements comportant au moins une chaîne grasse, de préférence choisis parmi les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, et leurs mélanges, de préférence la cétylhydroxyethylcellulose; de préférence le ou ledits polymères associatifs étant présent(s) en une teneur totale allant de 0,01 et 10%, et encore plus préférentiellement entre 0,05 et 5% du poids total de la composition, mieux entre 0,1 et 2% en poids par rapport au poids total de la composition de la composition colorante (A).

9. Dispositif selon l'une quelconque des revendications précédentes, **caractérisé en ce que** la composition colorante (A) comprend un ou plusieurs polymère(s) cationique(s) choisis parmi :

(1) les cyclopolymères d'alkyl diallyl amine ou de dialkyl diallyl ammonium tels que les homopolymères ou copolymères comportant comme constituant principal de la chaîne des motifs répondant aux formules (I) ou (II) :

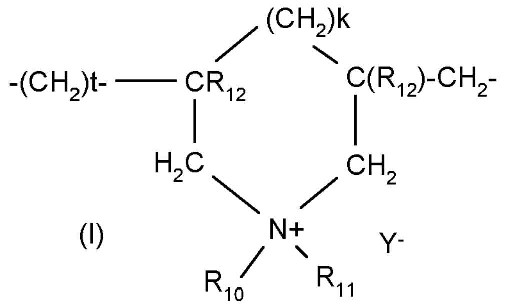

(I)

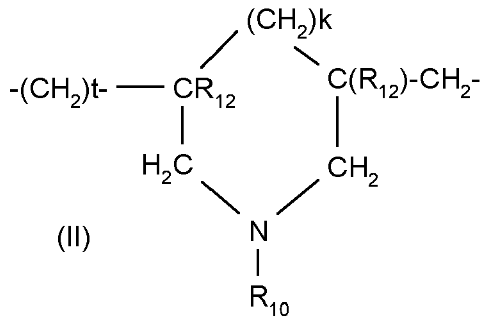

(II)

dans lesquelles

- k et t sont égaux à 0 ou 1, la somme k + t étant égale à 1 ;
- R12 désigne un atome d'hydrogène ou un radical méthyle ;
- R10 et R11, indépendamment l'un de l'autre, désignent un groupement alkyle en C1-C6, un groupement hydroxyalkyle en C1-C5, un groupement amidoalkyle en C1-C4; ou bien R10 et R11 peuvent désigner conjointement avec l'atome d'azote auquel ils sont rattachés, un groupement hétérocyclique tel que pipéridinyle ou morpholinyle; R10 et R11 indépendamment l'un de l'autre, désignent de préférence un groupement alkyle en C1-C4;
- $Y^-$ est un anion tel que bromure, chlorure, acétate, borate, citrate, tartrate bisulfate, bisulfite, sulfate, phosphat ; et/ou

(2) les polymères de diammonium quaternaire comprenant des motifs récurents de formule :

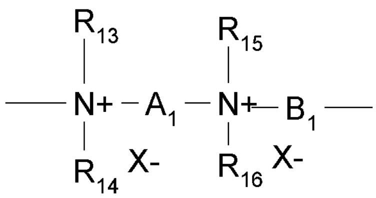

(III)

dans laquelle :

- R13, R14, R15 et R16, identiques ou différents, représentent des radicaux aliphatiques, alicycliques, ou arylaliphatiques comprenant de 1 à 20 atomes de carbone ou des radicaux hydroxyalkylaliphatiques en C1-C12, ou bien R13, R14, R15 et R16, ensemble ou séparément, constituent avec les atomes d'azote auxquels ils sont rattachés des hétérocycles comprenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
- A1 et B1 représentent des groupements divalents polyméthyléniques comprenant de 2 à 20 atomes de carbone, linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
- $X^-$ désigne un anion dérivé d'un acide minéral ou organique;

étant entendu que A1, R13 et R15 peuvent former avec les deux atomes d'azote auxquels ils sont rattachés un cycle pipérazinique ;

en outre si A1 désigne un radical alkylène ou hydroxyalkylène linéaire ou ramifié, saturé ou insaturé, B1 peut également désigner un groupement (CH2)n-CO-D-OC-(CH2)p-, avec n et p, identiques ou différents, étant des entiers variant de 2 à 20, et D désignant :

a) un reste de glycol de formule -O-Z-O-, où Z désigne un radical hydrocarboné linéaire ou ramifié ou un groupement répondant à l'une des formules suivantes: -(CH2CH2O)x-CH2CH2- et -[CH2CH(CH3)O]y-CH2CH(CH3)- où x et y désignent un nombre entier de 1 à 4, représentant un degré

de polymérisation défini et unique ou un nombre quelconque de 1 à 4 représentant un degré de polymérisation moyen ;
b) un reste de diamine bis-secondaire tel qu'un dérivé de pipérazine;
c) un reste de diamine bis-primaire de formule -NH-Y-NH- où Y désigne un radical hydrocarboné linéaire ou ramifié, ou bien le radical divalent -CH2-CH2-S-S-CH2-CH2- ;
d) un groupement uréylène de formule -NH-CO-NH- .

De préférence, $X^-$ est un anion tel que le chlorure ou le bromure. Ces polymères ont une masse molaire moyenne en nombre (Mn) généralement comprise entre 1000 et 100000.

**10.** Dispositif selon l'une quelconque des revendications 8 et 9, **caractérisé en ce que** le ou les polymère(s) cationique(s) est/sont choisi(s) parmi :

- (1) les homopolymères de dialkyl diallyl ammonium, de préférence les homopolymères de diméthyldiallylammonium, ; et/ou
- (2) les polymères cationiques qui sont constitués de motifs récurrents répondant à la formule :

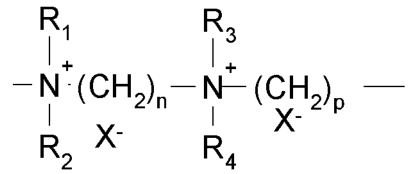
(IV)

dans laquelle R1, R2, R3 et R4, identiques ou différents, désignent un radical alkyle ou hydroxyalkyle ayant de 1 à 4 atomes de carbone, n et p sont des nombres entiers variant de 2 à 20, et X- est un anion dérivé d'un acide minéral ou organique ; de préférence le polymère composé de motifs formule (IV) pour lequel R1, R2, R3 et R4 représentent un radical méthyle, n=3, p=6 et X = Cl, dénommé Hexadimethrine chloride .

**11.** Dispositif selon l'une quelconque des revendications précédentes, **caractérisé en ce que** la composition (B) comprend une ou plusieurs gommes de scléroglucane, de préférence la ou les gommes de scléroglucane représentant de 0,5 à 10 % en poids, plus préférentiellement de 0,5 à 5 % en poids, et encore plus préférentiellement de 0,5 à 3 % en poids, encore plus préférentiellement et encore plus préférentiellement de 0,5 à 2 %, voire de 0,7 à 2% en poids, par rapport au poids total de la composition oxydante (B)

**12.** Dispositif selon la revendication précédente **caractérisé en ce que** le ou les agents oxydants chimique sont choisis parmi le peroxyde d'hydrogène et/ou un ou plusieurs système(s) générateur(s) de peroxyde d'hydrogène, de préférence parmi le peroxyde d'hydrogène.

**13.** Dispositif selon l'une quelconque des revendications précédentes constitué d'un récipient pressurisé par un ou plusieurs gaz propulseurs.

**14.** Dispositif selon la revendication 15 dans lequel les compartiments (a) et (b) sont des poches flexibles, de préférence en matériau métallique ou plastique, et le ou les gaz propulseurs se trouvent dans le volume défini entre les parois du récipient et les poches flexibles

**15.** Procédé de coloration des fibres kératiniques, de préférence des fibres kératiniques humaines telles que les cheveux, mettant en oeuvre l'application sur les fibres :

a) d'une composition colorante (A) telle que définie selon l'une quelconque de revendications précédentes ; et
b) d'une composition oxydante (B) telle que définie selon l'une quelconque de revendications précédentes.

les compositions (A) et (B) étant conditionnées dans un dispositif constitué d'un récipient, de préférence pressurisé, comprenant au moins deux compartiments (a) et (b) séparés l'un de l'autre, le compartiment (a) comprenant la composition colorante **(**A**)** et le compartiment (b) comprenant la composition oxydante **(**B**)**, le dispositif comprenant un moyen de distribution muni d'au moins un orifice de distribution, en communication avec les compartiments (a) et (b), permettant de délivrer les compositions **(**A**)** et **(**B**)** de manière simultanée.

## Patentansprüche

1. Vorrichtung zum Abgeben eines Produkts zum Färben von Keratinfasern, bestehend aus einen Behälter, der Folgendes umfasst:

   i) mindestens zwei voneinander getrennten Kompartimenten (a) und (b),

      a) wobei Kompartiment a eine Zusammensetzung (A) umfasst, umfassend:

         - - einen oder mehrere Oxidationsfarbstoff(e);
         - - ein oder mehrere alkalische Mittel,
         wobei das oder die alkalische (n) Mittel aus Ammoniak, Alkalimetall- oder Erdalkalimetallmetasilikaten, Alkanolaminen, Aminosäuren und deren Gemischen ausgewählt ist bzw. sind, und

      b) Kompartiment b eine oxidierende Zusammensetzung B umfasst, umfassend:

         - ein oder mehrere chemische(s) Oxidationsmittel, das bzw. die vorzugsweise aus Wasserstoffperoxid und/oder einem oder mehreren Wasserstoffperoxid erzeugenden System(en) ausgewählt ist bzw. sind, und
         - die eine und/oder die andere der Zusammensetzungen (A) und/oder (B) ein oder mehrere Scleroglucan-Gummi(s) umfasst;

   ii) ein Abgabemittel, das mit mindestens einer Abgabeöffnung in Kommunikation mit den Kompartimenten (a) und (b) ausgestattet ist, was es gestattet, die Zusammensetzungen A und B gleichzeitig, im gemischten oder getrennten Zustand, abzugeben.

2. Vorrichtung nach dem vorhergehenden Anspruch, **dadurch gekennzeichnet, dass** die Zusammensetzung (A) ein oder mehrere Scleroglucan-Gummis, vorzugsweise in einem Gesamtgehalt von mehr als oder gleich 0,5 Gew.-%, bezogen auf das Gesamtgewicht der Zusammensetzung (A), umfasst und die Zusammensetzung (B) ein oder mehrere Scleroglucan-Gummis, vorzugsweise in einem Gesamtgehalt von mehr als oder gleich 0,5 Gew.-%, bezogen auf das Gesamtgewicht der Zusammensetzung (B), umfasst.

3. Vorrichtung nach einem der vorhergehenden Ansprüche, **dadurch gekennzeichnet, dass** das oder die Scleroglucan-Gummis 0,5 bis 10 Gew.-%, stärker bevorzugt 0,5 bis 5 Gew.-% und noch stärker bevorzugt 0,5 bis 3 Gew.-%, besser 0,5 bis 2 Gew.-% und noch stärker bevorzugt 0,7 bis 1,5 Gew.-%, bezogen auf das Gesamtgewicht der Zusammensetzung (A), ausmachen.

4. Vorrichtung nach einem der vorhergehenden Ansprüche, **dadurch gekennzeichnet, dass** der oder die Oxidationsfarbstoff(e) aus Benzol-Oxidationsbasen ausgewählt ist bzw. sind, die vorzugsweise aus paraPhenylendiaminen, Bis(phenyl)alkylendiaminen, para-Aminophenolen, ortho-Aminophenolen, heterozyklischen Basen und deren Additionssalzen ausgewählt sind, gegebenenfalls in Kombination mit einem oder mehreren Kupplern, die vorzugsweise aus meta-Phenylendiaminen, meta-Aminophenolen, meta-Diphenolen, Naphthalin-Kupplern, heterozyklischen Kupplern sowie deren Additionssalzen ausgewählt sind.

5. Vorrichtung nach einem der vorhergehenden Ansprüche, **dadurch gekennzeichnet, dass** das oder die alkalische(n) Mittel aus anorganischen und/oder organischen alkalischen Mitteln, vorzugsweise aus einem Gemisch mindestens eines anorganischen alkalischen Mittels und mindestens eines organischen alkalischen Mittels, ausgewählt ist bzw. sind.

6. Vorrichtung nach einem der vorhergehenden Ansprüche, **dadurch gekennzeichnet, dass** die Zusammensetzung Ammoniak (Ammoniumhydroxid) und ein oder mehrere Alkanolamin(e), vorzugsweise Monoethanolamin, umfasst.

7. Vorrichtung nach einem der vorhergehenden Ansprüche, **dadurch gekennzeichnet, dass** die Färbezusammensetzung (A) ein oder mehrere Tenside umfasst, die vorzugsweise aus kationischen und/oder nichtionischen Tensiden ausgewählt sind, wobei das oder die Tensid(e) vorzugsweise in einem Gesamtgehalt im Bereich von 0,01 bis 20 Gew.-%, stärker bevorzugt von 0,05 bis 10 Gew.-%, besser von 0,1 bis 5 Gew.-%, bezogen auf das Gesamtgewicht der Färbezusammensetzung (A), vorhanden ist bzw. sind.

**8.** Vorrichtung nach einem der vorhergehenden Ansprüche, **dadurch gekennzeichnet, dass** die Färbezusammensetzung (A) ein oder mehrere, vorzugsweise nichtionische(s), assoziative(s) Polymer(e) enthält, die aus Cellulosen ausgewählt sind, die durch mindestens eine Fettkette tragende Gruppen modifiziert sind, vorzugsweise aus Hydroxyethylcellulosen ausgewählt sind, die durch Gruppen modifiziert sind, die mindestens eine Fettkette tragen, wie Alkyl-, Arylalkyl-, Alkylarylgruppen oder deren Gemische und wobei die Alkylgruppen vorzugsweise $C_8$-$C_{22}$ und deren Gemische sind, vorzugsweise Cetylhydroxyethylcellulose, wobei vorzugsweise das oder die assoziative(n) Polymer(e) in einem Gesamtgehalt im Bereich von 0,01 Gew.-% bis 10 Gew.-% und noch stärker bevorzugt zwischen 0,05 und 5 Gew.-%, bezogen auf das Gesamtgewicht der Zusammensetzung, besser zwischen 0,1 und 2 Gew.-%, bezogen auf das Gesamtgewicht der Zusammensetzung der Färbezusammensetzung (A), vorhanden ist bzw. sind.

**9.** Vorrichtung nach einem der vorhergehenden Ansprüche, **dadurch gekennzeichnet, dass** die Färbezusammensetzung (A) ein oder mehrere kationische(s) Polymer(e) umfasst, ausgewählt aus:

(1) Cyclopolymeren von Alkyldiallylamin oder Dialkyldiallylammonium, wie Homopolymere oder Copolymere, die als Hauptbestandteil der Kette Einheiten entsprechend den Formeln (I) oder (II) enthalten:

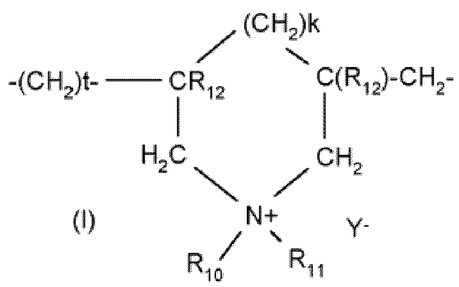
(I)

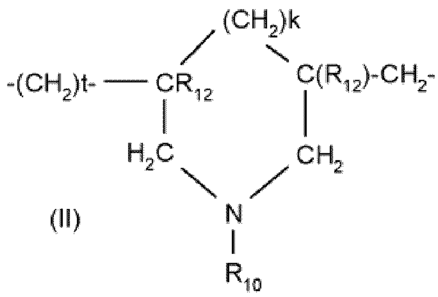
(II)

,

in denen

- k und t gleich 0 oder 1 sind, wobei die Summe k + t gleich 1 ist;
- R12 ein Wasserstoffatom oder einen Methylrest bezeichnet;
- R10 und R11 unabhängig voneinander eine C1-C6-Alkylgruppe, eine C1-C5-Hydroxyalkylgruppe, eine C1-C4-Amidoalkylgruppe bezeichnen oder auch R10 und R11 zusammen mit dem Stickstoffatom, an das sie gebunden sind, eine heterocyclische Gruppe, wie Piperidinyl oder Morpholinyl, bezeichnen können; R10 und R11 unabhängig voneinander vorzugsweise eine C1-C4-Alkylgruppe bezeichnen;
- $Y^-$ ein Anion ist, wie Bromid, Chlorid, Acetat, Borat, Citrat, Tartrat, Bisulfat, Bisulfit, Sulfat oder Phosphat; und/oder

(2) quartäres-Diammonium-Polymeren, die sich wiederholende Einheiten der folgenden Formel umfassen:

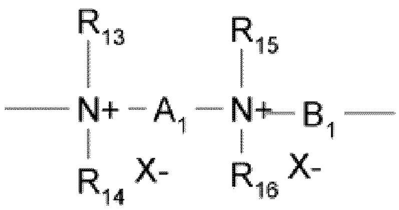
(III)

,

worin:

- R13, R14, R15 und R16, die gleich oder verschieden sind, aliphatische, alicyclische oder arylaliphatische Reste, die 1 bis 20 Kohlenstoffatome umfassen, oder aliphatische C1-C12-Hydroxyalkylreste darstellen

oder auch R13, R14, R15 und R16, zusammen oder getrennt, mit den Stickstoffatomen, an die sie gebunden sind, Heterocyclen bilden, die gegebenenfalls ein zweites, anderes Heteroatom als Stickstoff umfassen

oder auch R13, R14, R15 und R16 einen geraden oder verzweigten C1-C6-Alkylrest darstellen, der mit einer Nitril-, Ester-, Acyl-, Amidgruppe oder -CO-O-R17-D oder -CO-NH-R17-D substituiert ist, wobei R17 ein Alkylen ist und D eine quartäre Ammoniumgruppe ist;

- A1 und B1 gerade oder verzweigte, gesättigte oder ungesättigte, zweiwertige Polymethylengruppen darstellen, die 2 bis 20 Kohlenstoffatome umfassen und die, gebunden oder in die Hauptkette interkaliert, ein oder mehrere aromatische Ringe oder ein oder mehrere Sauerstoff-, Schwefelatome oder Sulfoxid-, Sulfon-, Disulfid-, Amino-, Alkylamino-, Hydroxyl-, quartäre Ammonium-, Ureido-, Amid- oder Estergruppen enthalten können, und
- $X^-$ ein von einer anorganischen oder organischen Säure stammendes Anion bezeichnet;

wobei selbstverständlich ist, dass A1, R13 und R15 mit den beiden Stickstoffatomen, an die sie gebunden sind, einen Piperazinring bilden können;

wobei außerdem, wenn A1 einen geraden oder verzweigten, gesättigten oder ungesättigten Alkylen- oder Hydroxyalkylenrest bezeichnet, B1 auch eine Gruppe (CH2)n-CO-D-OC-(CH2)p- bezeichnen kann, wobei n und p, die gleich oder verschieden sind, ganze Zahlen im Bereich von 2 bis 20 sind und D Folgendes bezeichnet:

a) einen Glykolrest der Formel -O-Z-O-, wobei Z einen geraden oder verzweigten Kohlenwasserstoffrest oder eine Gruppe bezeichnet, die einer der folgenden Formeln entspricht: -(CH2CH2O)x-CH2CH2- und -[CH2CH(CH3)O]y-CH2CH(CH3)-, wobei x und y eine ganze Zahl von 1 bis 4, die einen definierten und einzigen Polymerisationsgrad darstellt, oder eine beliebige Zahl von 1 bis 4 bezeichnen, die einen mittleren Polymerisationsgrad darstellt;
b) einen bis-sekundäres-Diamin-Rest, wie ein Piperazinderivat;
c) einen bis-primäres-Diamin-Rest der Formel -NH-Y-NH-, wobei Y einen geraden oder verzweigten Kohlenwasserstoffrest oder auch den zweiwertigen Rest - CH2-CH2-S-S-CH2CH2- bezeichnet;
d) eine Ureylengruppe der Formel -NH-CO-NH-;

vorzugsweise ist $X^-$ ein Anion, wie ein Chlorid oder Bromid; diese Polymere haben im Allgemeinen eine zahlenmittlere Molekülmasse (Mn) zwischen 1000 und 100000.

**10.** Vorrichtung nach einem der Ansprüche 8 und 9, **dadurch gekennzeichnet, dass** das das oder die kationische(n) Polymer(e) aus den Folgenden ausgewählt ist bzw. sind:

- (1) Dialkyldiallylammonium-Homopolymeren, vorzugsweise Dimethyldiallylammonium-Homopolymeren, und/oder
- (2) kationischen Polymeren, die aus sich wiederholenden Einheiten entsprechend der folgenden Formel bestehen:

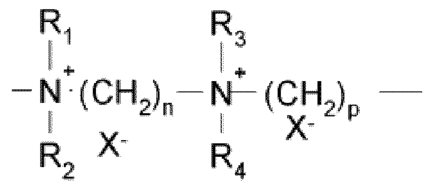

(IV)

,

worin R1, R2, R3 und R4, die gleich oder verschieden sind, einen Alkyl- oder Hydroxyalkylrest mit 1 bis 4 Kohlenstoffatomen bezeichnen, n und p ganze Zahlen im Bereich von 2 bis 20 sind und $X^-$ ein von einer anorganischen oder organischen Säure stammendes Anion ist, vorzugsweise das aus Motiven der Formel (IV) bestehende Polymer, in dem R1, R2, R3 und R4 einen Methylrest darstellen, n=3, p=6 und X = Cl, das als Hexadimethrinchlorid bezeichnet wird.

**11.** Vorrichtung nach einem der vorhergehenden Ansprüche, **dadurch gekennzeichnet, dass** die Zusammensetzung (B) einen oder mehrere Scleroglucan-Gummi(s) umfasst, wobei vorzugsweise der oder die Scleroglucan-Gummi(s) 0,5 bis 10 Gew.-%, stärker bevorzugt 0,5 bis 5 Gew.-% und noch stärker bevorzugt 0,5 bis 3 Gew.-%, noch stärker bevorzugt und noch stärker bevorzugt 0,5 bis 2 Gew.-% oder sogar 0,7 bis 2 Gew.-%, bezogen auf das Gesamtgewicht der oxidierenden Zusammensetzung (B), ausmacht bzw. ausmachen.

12. Vorrichtung nach dem vorhergehenden Anspruch, **dadurch gekennzeichnet, dass** das oder die chemische(n) Oxidationsmittel aus Wasserstoffperoxid und/oder einem oder mehreren Wasserstoffperoxid erzeugenden System(en), vorzugsweise aus Wasserstoffperoxid, ausgewählt ist bzw. sind.

13. Vorrichtung nach einem der vorhergehenden Ansprüche, die aus einem durch ein oder mehrere Treibgas(e) unter Druck gesetzten Behälter besteht.

14. Vorrichtung nach Anspruch 15, wobei die Kompartimente (a) und (b) flexible Beutel, vorzugsweise aus Metall- oder Kunststoffmaterial, sind und das oder die Treibgas(e) sich in einem zwischen den Wänden des Behälters und den flexiblen Beuteln definierten Volumen befindet bzw. befinden.

15. Verfahren zum Färben von Keratinfasern, vorzugsweise menschlichen Keratinfasern, das das Auftragen von Folgendem auf die Fasern vorsieht:

a) einer Färbezusammensetzung (A) nach einem der vorhergehenden Ansprüche und
b) einer oxidierenden Zusammensetzung (B) nach einem der vorhergehenden Ansprüche,

wobei die Zusammensetzungen (A) und (B) in einer Vorrichtung abgepackt sind, die aus einem, vorzugsweise unter Druck stehenden, Behälter besteht, der mindestens zwei voneinander getrennte Kompartimente (a) und (b) umfasst, wobei das Kompartiment (a) die Färbezusammensetzung (A) umfasst und das Kompartiment (b) die oxidierende Zusammensetzung (B) umfasst, wobei die Vorrichtung ein Abgabemittel umfasst, das mit mindestens einer Abgabeöffnung in Kommunikation mit den Kompartimenten (a) und (b) ausgestattet ist, was es gestattet, die Zusammensetzungen (A) und (B) gleichzeitig abzugeben.

## Claims

1. Device for dispensing a product for dyeing keratin fibers, consisting of a container comprising:

i) at least two compartments (a) and (b) which are separate from each other,

a) compartment a comprising a composition (A) comprising:

- one or more oxidation dyes;
- one or more alkaline agents; wherein the alkaline agent(s) are chosen from aqueous ammonia, alkali metal or alkaline-earth metal metasilicates, alkanolamines, amino acids, and mixtures thereof; and

b) compartment b comprising an oxidizing composition B comprising:

- one or more chemical oxidizing agents, preferably chosen from hydrogen peroxide and/or one or more systems for generating hydrogen peroxide; and

- one and/or the other of compositions (A) and/or (B) comprising one or more scleroglucan gums;

ii) a dispensing means equipped with at least one dispensing orifice, in communication with compartments (a) and (b), for simultaneously dispensing compositions A and B, in mixed or separate form.

2. Device according to the preceding claim, **characterized in that** composition (A) comprises one or more scleroglucan gums, preferably in a total weight content of greater than or equal to 0.5% relative to the total weight of composition (A), and composition (B) comprises one or more scleroglucan gums, preferably in a total weight content of greater than or equal to 0.5% relative to the total weight of composition (B).

3. Device according to either of the preceding claims, **characterized in that** the scleroglucan gum(s) represent from 0.5% to 10% by weight, more preferentially from 0.5% to 5% by weight, and even more preferentially from 0.5% to 3% by weight, better still from 0.5% to 2% by weight and even more preferentially from 0.7% to 1.5% by weight, relative to the total weight of composition (A).

4. Device according to any one of the preceding claims, **characterized in that** the oxidation dye(s) are chosen from

benzene-based oxidation bases, preferably chosen from para-phenylenediamines, bis(phenyl)alkylenediamines, para-aminophenols, orthoaminophenols, heterocyclic bases and the addition salts thereof; optionally combined with one or more couplers preferably chosen from meta-phenylenediamines, meta-aminophenols, meta-diphenols, naphthalene-based couplers, and heterocyclic couplers, and also the addition salts thereof.

**5.** Device according to any one of the preceding claims, **characterized in that** the alkaline agent(s) are chosen from mineral and/or organic alkaline agents, preferably from a mixture of at least one mineral alkaline agent and of at least one organic alkaline agent.

**6.** Device according to any one of the preceding claims, **characterized in that** the composition comprises aqueous ammonia (ammonium hydroxide) and one or more alkanolamines, preferably monoethanolamine.

**7.** Device according to any one of the preceding claims, **characterized in that** the dye composition (A) comprises one or more surfactants, preferably chosen from cationic and/or nonionic surfactants; said surfactant(s) are preferably present in a total content ranging from 0.01% to 20%, more preferentially from 0.05% to 10% by weight and better still from 0.1% to 5% by weight relative to the total weight of the dye composition (A).

**8.** Device according to any one of the preceding claims, **characterized in that** the dye composition (A) comprises one or more associative polymers, which are preferably nonionic, chosen from celluloses modified with groups including at least one fatty chain, preferably chosen from hydroxyethylcelluloses modified with groups including at least one fatty chain such as alkyl, arylalkyl and alkylaryl groups, or mixtures thereof, and in which the alkyl groups are preferably $C_8$-$C_{22}$, and mixtures thereof, preferably cetylhydroxyethylcellulose; said associative polymer(s) preferably being present in a total content ranging from 0.01% to 10%, and even more preferentially between 0.05% and 5% of the total weight of the composition, better still between 0.1% and 2% by weight relative to the total weight of the composition of the dye composition (A).

**9.** Device according to any one of the preceding claims, **characterized in that** the dye composition (A) comprises one or more cationic polymers chosen from:

(1) cyclopolymers of alkyldiallylamine or of dialkyldiallylammonium, such as the homopolymers or copolymers including, as main constituent of the chain, units corresponding to formula (I) or (II):

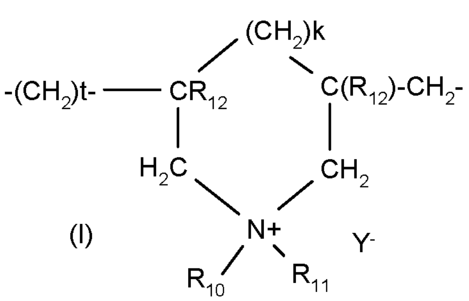
(I)

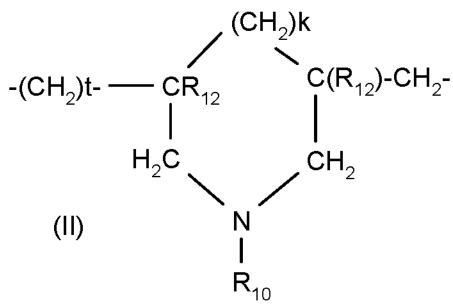
(II)

in which

- k and t are equal to 0 or 1, the sum k + t being equal to 1;
- R12 denotes a hydrogen atom or a methyl radical;
- R10 and R11, independently of each other, denote a C1-C6 alkyl group, a C1-C5 hydroxyalkyl group, a C1-C4 amidoalkyl group; or alternatively R10 and R11 may denote, together with the nitrogen atom to which they are attached, a heterocyclic group such as piperidyl or morpholinyl; R10 and R11, independently of each other, preferably denote a C1-C4 alkyl group;
- $Y^-$ is an anion such as bromide, chloride, acetate, borate, citrate, tartrate, bisulfate, bisulfite, sulfate, phosphate; and/or

(2) quaternary diammonium polymers comprising repeating units of formula:

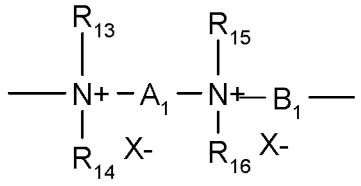 (III)

in which:

- R13, R14, R15 and R16, which may be identical or different, represent aliphatic, alicyclic or arylaliphatic radicals comprising from 1 to 20 carbon atoms or C1-C12 hydroxyalkyl aliphatic radicals,

  or else R13, R14, R15 and R16, together or separately, form, with the nitrogen atoms to which they are attached, heterocycles optionally comprising a second non-nitrogen heteroatom;
  or else R13, R14, R15 and R16 represent a linear or branched C1-C6 alkyl radical substituted with a nitrile, ester, acyl, amide or -CO-O-R17-D or -CO-NH-R17-D group, where R17 is an alkylene and D is a quaternary ammonium group;

- A1 and B1 represent linear or branched, saturated or unsaturated, divalent polymethylene groups comprising from 2 to 20 carbon atoms, which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
- $X^-$ denotes an anion derived from a mineral or organic acid;
it being understood that A1, R13 and R15 can form, with the two nitrogen atoms to which they are attached, a piperazine ring;
in addition, if A1 denotes a linear or branched, saturated or unsaturated alkylene or hydroxyalkylene radical, B1 may also denote a group (CH2)n-CO-D-OC-(CH2)p- with n and p, which may be identical or different, being integers ranging from 2 to 20, and D denoting:

  a) a glycol residue of formula -O-Z-O-, in which Z denotes a linear or branched hydrocarbon-based radical or a group corresponding to one of the following formulae: - (CH2CH2O)x-CH2CH2- and -[CH2CH(CH3)O]y-CH2CH(CH3)-, in which x and y denote an integer from 1 to 4, representing a defined and unique degree of polymerization or any number from 1 to 4 representing an average degree of polymerization;
  b) a bis-secondary diamine residue, such as a piperazine derivative;
  c) a bis-primary diamine residue of formula -NH-Y-NH-, in which Y denotes a linear or branched hydrocarbon-based radical, or else the divalent radical -CH2-CH2-S-S-CH2-CH2-;
  d) a ureylene group of formula -NH-CO-NH-.

Preferably, $X^-$ is an anion, such as chloride or bromide. These polymers have a numberaverage molar mass (Mn) generally of between 1000 and 100 000.

**10.** Device according to either of Claims 8 and 9, **characterized in that** the cationic polymer(s) are chosen from:

- (1) dialkyldiallylammonium homopolymers, preferably dimethyldiallylammonium homopolymers; and/or
- (2) cationic polymers that are constituted of repeating units corresponding to the formula:

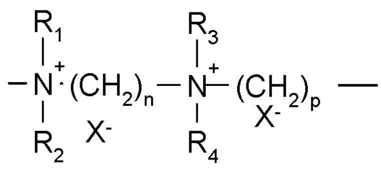 (IV)

in which R1, R2, R3 and R4, which may be identical or different, denote an alkyl or hydroxyalkyl radical containing from 1 to 4 carbon atoms, n and p are integers ranging from 2 to 20, and X- is an anion derived from a mineral or organic acid; preferably, the polymer composed of units of formula (IV) for which R1, R2, R3 and R4 represent a methyl radical, n = 3, p = 6 and X = Cl, known as hexadimethrine chloride.

**11.** Device according to any one of the preceding claims, **characterized in that** composition (B) comprises one or more scleroglucan gums, the scleroglucan gum(s) preferably representing from 0.5% to 10% by weight, more preferentially from 0.5% to 5% by weight, and even more preferentially from 0.5% to 3% by weight, even more preferentially and even more preferentially from 0.5% to 2%, or even from 0.7% to 2% by weight, relative to the total weight of the oxidizing composition (B).

**12.** Device according to the preceding claim, **characterized in that** the chemical oxidizing agent(s) are chosen from hydrogen peroxide and/or one or more systems for generating hydrogen peroxide, preferably from hydrogen peroxide.

**13.** Device according to any one of the preceding claims, consisting of a container pressurized with one or more propellant gases.

**14.** Device according to Claim 15, in which compartments (a) and (b) are flexible pouches, preferably made of metal or plastic, and the propellant gas(es) are in the volume defined between the walls of the container and the flexible pouches.

**15.** Process for dyeing keratin fibers, preferably human keratin fibers such as the hair, involving the application to the fibers:

a) of a dye composition (A) as defined in any one of the preceding claims; and
b) of an oxidizing composition (B) as defined in any one of the preceding claims; compositions (A) and (B) being packaged in a device consisting of a container, preferably a pressurized container, comprising at least two compartments (a) and (b) which are separated from each other, compartment (a) comprising the dye composition (A) and compartment (b) comprising the oxidizing composition (B), the device comprising a dispensing means equipped with at least one dispensing orifice, in communication with compartments (a) and (b), for dispensing compositions (A) and (B) simultaneously.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 2010192969 A1, DEGEORGE MICHAEL
- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988
- FR 2886136 A
- FR 2633940